# EUROPEAN PATENT SPECIFICATION

(11) **EP 0 511 652 B1**
(45) Date of publication and mention of the grant of the patent: **29.11.1995**
(21) Application number: 92107311.0
(22) Date of filing: 29.04.1992
(51) Int. Cl.: A61K 7/06

(54) **Hair shampoo composition to impart improved hair conditioning properties**
Haarkonditioniershampoo
Shampooing pour le conditionnement des cheveux

(30) Priority: 29.04.1991 US 692709
(43) Date of publication of application: 04.11.1992
(73) Proprietor: HELENE CURTIS, INC., Chicago, Illinois 60610-4713 (US)
(72) Inventor: Duffy, Michele, Northbrook, IL (US); Bergmann, Wolfgang, Highland Park, IL (US)
(74) Representative: KUHNEN, WACKER & PARTNER

(56) References cited:
- EP-A- 0 117 135
- FR-A- 2 548 019
- WORLD PATENTS INDEX LATEST Week 8932, Derwent Publications Ltd., London, GB; AN 89-232269
- WORLD PATENTS INDEX LATEST Week 9038, Derwent Publications Ltd., London, GB; AN 90-287219

## Description

The present invention relates to a hair shampoo-conditioner composition and to a method of treating hair that cleanses the hair and imparts improved wet stage and dry stage conditioning properties to hair in a single application of the composition. More particularly, the present invention is directed to a hair shampoo-conditioner composition including: a) an anionic cleansing surfactant, like an alkyl ether sulfate, such as sodium lauryl ether sulfate; b) a polymeric cationic conditioning compound, such as a quaternized guar gum; c) a cationic conditioning surfactant, such as a quaternary ammonium compound including a fatty amidoalkyl substituent or a quaternized phosphate triester; and d) a fatty ester, in e) a suitable carrier. The hair shampoo-conditioner composition effectively cleanses the hair and simultaneously imparts unexpectedly improved wet stage and dry stage conditioning properties to hair in a single application of the composition. Surprisingly, a hair shampoo-conditioner composition of the present invention does not exhibit an interaction between the cationic components and the anionic components in the composition, and therefore the anionic components and the cationic components are available to effectively cleanse the hair and to impart wet stage and dry stage conditioning properties to the hair.

### BACKGROUND OF THE INVENTION

Most individuals buy and use a hair shampoo for its cleansing properties. Furthermore, in addition to clean hair, the consumer desires sufficiently-conditioned hair that holds a preset configuration. However, present-day hair shampoos generally are formulated with highly-effective synthetic surfactants, like anionic surfactants, that primarily clean, as opposed to conditioning, the hair. Therefore, it is not surprising that hair shampoos usually neither help detangle wet hair nor impart any residual hair conditioning benefits to dry hair, such as the manageability or styleability of hair sets.

Consequently, after shampooing, the hair normally is left in a cosmetically-unsatisfactory state because an anionic surfactant-based hair shampoo composition not only removes all of the dirt and soil from the hair, but also removes essentially all of the sebum that is naturally present on the surface of the hair fibers. Therefore, it was found that the properties of anionic surfactants that effectively cleanse the hair also serve to leave the hair in a cosmetically-unsatisfactory condition. In general therefore, shampooing the hair with a hair shampoo composition including anionic surfactants, or nonionic surfactants or amphoteric surfactants, leaves the hair, after rinsing with water, with an undesirable harsh, dull and dry touch or feel, usually called "creak".

As a result, thoroughly cleansed hair is extremely difficult to comb, in either the wet or dry stage, because the individual hair fibers tend to snarl, kink, and interlock with each other. In addition, incompletely dried hair, such as hair dried with a towel, has poor brushing properties. Then, after complete drying, the hair does not set well. Furthermore, the combing or brushing property of the dried hair remains poor. The dried hair also has undesirable electrostatic properties in a low humidity atmosphere that cause the hair to "fly away", thereby further reducing the brushing property of the hair. The unsatisfactory combing or brushing property of freshly-shampooed hair also causes hair damage, such as split ends or hair breakage. In addition, the natural luster and resiliency of the hair is reduced.

Accordingly, freshly-shampooed hair usually requires a post-shampoo hair treatment with a conditioning composition to improve the unsatisfactory physical and cosmetic condition of the hair. A conditioning composition normally is applied separately from the hair shampoo, and usually is a rinse or a cream-like lotion containing a cationic compound. Therefore, investigators have sought hair shampoo compositions that both cleanse the hair and leave the hair in a cosmetically-satisfactory state, such that the subsequent treatment with a conditioner composition can be avoided.

Consequently, investigations were directed at providing a composition that behaves both as a shampoo and as a hair conditioner, but the resulting shampoo-conditioner compositions possessed several disadvantages. For example, it is known in the art that anionic surfactants are suitable for cleansing the hair, and that, in many instances, cationic surfactants and cationic polymers are suitable hair conditioners. However, the major difficulty encountered by investigators is the inherent incompatibility between an anionic surfactant and a cationic surfactant or cationic polymer. Consequently, contact between the anionic surfactant and the cationic surfactant or cationic polymer either produces an intractable precipitate that forms immediately, or causes an interaction between the anionic and cationic components that significantly reduces their respective cleansing and conditioning properties. The reduction in cleansing and conditioning effectiveness also is observed in compositions wherein the anionic and cationic components do not precipitate from the composition but remain in solution or in a suspended state. This incompatibility between an anionic compound and a cationic compound is well recognized by workers skilled in the art. For example, Sagarin in Cosmetics, Interscience Publishers, Inc., New York, p. 538, 1957, states that anionic and cationic compounds cannot be used in combination because they react to form insoluble salts. Thus, in practice, consumer needs traditionally have been met by applying a nonsubstantive, anionic surfactant-based shampoo to the hair to cleanse the hair, then rinsing the hair, followed by applying a conditioner composition including a substantive cationic compound to the hair to condition the hair.

As previously discussed, freshly-shampooed hair, being inclined to knot and tangle, is difficult to manage and comb. The wet combing problem has been solved by treating freshly-shampooed hair with a conditioner composition that includes a compound to coat the hair shaft and cause the individual hair shafts to resist tangling and matting because of conditioner compound residue on the hair shaft. Until recently the desirable properties of both a hair shampoo and a hair conditioner composition could not be incorporated into a single composition. Therefore, a shampoo composition and a conditioner composition were applied sequentially to achieve the benefits provided by each composition. Investigators attempting to combine all, or some, of the beneficial properties of a shampoo composition and of a conditioner composition into a single shampoo-conditioner composition concentrated particularly on incorporating the properties of a post-shampoo conditioning rinse into a hair shampoo composition.

Therefore, because hair shampoo compositions are predominantly anionic in character, the incorporation of a substantive cationic compound into an anionic shampoo composition ranges from difficult to impossible because of the inherent incompatibility between anionic and cationic surfactants. Nevertheless, a combination shampoo-conditioner composition is desirable because of the convenience such a combination product offers to the consumer. In such a shampoo-conditioner product, the anionic surfactant acts to rid the hair and scalp of dirt, surface film, debris, and the like, while the cationic compound deposits on the hair to provide conditioning benefits, such as manageability, shine and texture. However, until the composition and method of the present invention, it has proven very difficult to provide a stable hair shampoo-conditioner composition because of the inherent incompatibility between cationic and anionic surfactants. Consequently, and in accordance with an important feature of the present invention, a polymeric cationic conditioning compound and a cationic conditioning surfactant are incorporated into a composition wherein an interaction between the anionic and cationic components of the composition is essentially precluded. The hair shampoo-conditioner then is utilized to clean the hair and, simultaneously, to impart conditioning properties to the hair.

Therefore, the need for an effective and stable shampoo-conditioner composition that cleanses the hair and conditions the hair, i.e., renders the hair more manageable, in a single hair treatment has long been recognized in the art. Accordingly, although conditioning compositions for application to previously-shampooed hair are well known, only recently have shampoo-conditioner compositions become available. For example, some shampoo-conditioner compositions are specially formulated for mildness, and accordingly low detergency, in order to leave a portion of the natural oils on the hair shaft. However, hair treated with this type of composition becomes greasy, dirty looking and dirty feeling relatively quickly.

Another type of shampoo-conditioner composition includes an oily component, such as a polyglycol, a glycol ester of a fatty acid, a natural or synthetic wax or a lanolin derivative, that is deposited on the hair during shampooing. However, the oily nature of such components reduces shampoo lathering and contributes to the feeling of greasy, dirty hair relatively soon after shampooing. Another type of shampoo-conditioner composition includes a substantive cationic polymer that deposits on the hair shaft during shampooing to impart the desired degree of manageability. However, the cationic polymer also gave the hair a greasy feeling as described above. The primary difficulty encountered in preparing this type of shampoo-conditioner composition has been achieving a stable composition without destroying the delicate balance of conditioning, cleansing, consumer appeal, esthetic properties and other functional properties. Surprisingly and unexpectedly, although the composition of the present invention includes both a polymeric cationic conditioning compound and an oily compound, i.e. a fatty ester, the composition is sufficiently stable, lathers sufficiently, cleanses the hair and imparts conditioning properties to the hair without a greasy feeling, while maintaining excellent physical and esthetic properties for consumer appeal.

Therefore, the present invention relates to a shampoo-conditioner composition for cleansing the hair and for imparting improved physical and cosmetic properties to the hair, such as improved combing properties, luster and manageability. It is known that anionic surfactants are suitable for shampooing the hair, and that cationic surfactants and cationic polymers are useful for conditioning the hair. In addition, combining an anionic surfactant and a cationic surfactant in a shampoo-conditioner composition has proven difficult, to impossible, because of the inherent chemical incompatibility between the two classes of surfactants. Consequently, and in accordance with the present invention, it has been found that anionic surfactants can be combined with a polymeric cationic conditioning compound and a cationic conditioning surfactant to provide a stable and effective hair shampoo-conditioner composition. As manufactured, the composition is metastable, wherein the term "metastable composition" is defined as a composition that is sufficiently stable to resist phase separation during storage and essentially precludes an interaction between the cationic and anionic components of the composition; but, upon application to the hair, deposits a substantial amount of the cationic and fatty ester conditioning components onto the hair shaft that withstand rinsing from the hair during the shampooing and rinsing process. It also has been found that the optional addition of other conditioning agents, like nonionic conditioning agents, to the shampoo-conditioner composition of the present invention further improves the conditioning properties imparted to the treated hair.

More particularly, it has been found that a polymeric cationic conditioning compound and a cationic conditioning surfactant, when incorporated into a shampoo composition including a nonsubstantive anionic cleansing surfactant and a fatty ester, provide a hair shampoo-conditioner composition that generates sufficient lathering and thoroughly cleanses the hair, in addition to depositing a sufficient amount of the fatty ester, the cationic polymer and the cationic surfactant onto the hair to condition the hair without imparting a greasy feel to the hair. Such results are unexpected in the art because anionic surfactants, as a class, are essentially incompatible with a cationic polymer and cationic surfactant. Therefore, the compatibility demonstrated by the combination of the anionic cleansing surfactant and the cationic conditioning surfactant and the cationic conditioning polymers utilized in the present invention is both new and surprising, thereby permitting sufficient deposition of the substantive cationic conditioning components onto the hair, while the anionic cleansing surfactant shampoos the hair.

Overall, therefore, cationic compounds, such as cationic surfactants and cationic polymers, are known to be substantive to human hair and traditionally are used to complete the hair cleansing and hair conditioning cycle. The ability of a cationic compound to interact with the keratinous material of hair makes cationic compounds the most widely used compounds to impart the desired physical and cosmetic conditioning properties, such as wet hair detangling and dry hair manageability, to hair. Most commonly, the cationic compounds are applied to freshly shampooed and rinsed hair from a post-shampoo conditioning rinse. More recently, investigators have incorporated the cationic compounds into anionic surfactant-based hair shampoos by carefully balancing the composition to reduce or avoid an interaction between the cationic compounds and the anionic surfactants.

Cationic compounds that have been incorporated into anionic hair shampoo compositions include, for example, water-soluble proteins or protein degradation products, or polycationic polymers, such as the amino polycarbamide resins of the type described in DE-OS No. 21 50 899; polycationic cellulose derivatives of the type described in U.S. Patent No. 3,816,616; or polycationic guar derivatives of the type described in U.S. Patent No. 4,292,212. Many other water-soluble polymers containing cationic or quaternary ammonium groups have been proposed for use in an anionic surfactant-based hair shampoo. However, even though particular cationic compounds, especially particular cationic polymers, are compatible with anionic surfactants, a disadvantage common to all cationic hair conditioning compounds is that at least a partially-reduced conditioning effect is observed when the cationic compound is included in an anionic surfactant-based hair shampoo. In contrast, and as will be demonstrated more fully hereinafter, the hair shampoo-conditioner compositions of the present invention effectively cleanse the hair and impart conditioning properties to hair equivalent to properties imparted by a premium, post-shampoo conditioner composition.

Accordingly, the present invention is directed to a hair shampoo-conditioner composition, including a nonsubstantive and high-foaming anionic cleansing surfactant and a combination of cationic and nonionic hair-conditioning components, that simultaneously cleanses the hair and imparts desirable physical and cosmetic properties to the hair. By treating the hair with the shampoo-conditioner composition of the present invention, the hair is combed easily when wet and the hair possesses satisfactory cosmetic properties when dry, including, in particular, elasticity, body, sheen and manageability. In contrast to the prior art, wherein cationic polymers were blended primarily with amphoteric surfactants, the hair shampoo-conditioner composition of the present invention includes a cationic polymer, a cationic surfactant, a fatty ester and an anionic surfactant to cleanse the hair and to impart conditioning properties to the hair. Therefore, the stability and incompatibility problems normally encountered when a cationic surfactant and anionic surfactant are present in the same composition have been overcome.

Previous attempts to provide a combination shampoo-conditioner composition include the disclosure of Goff in U.S. Patent No. 2,950,255, wherein relatively small, equimolar amounts of an anionic surfactant and a cationic surfactant are included in a hair shampoo primarily based on amphoteric and nonionic surfactants. Amphoteric and nonionic surfactants are mild detergents and are compatible with cationic surfactants, but are not as effective in cleansing the hair as anionic surfactants. Similarly, Anguillo et al., in U.S. Patent No. 3,816,616, discloses the use of a cationic polymer in an anionic surfactant-based shampoo to provide a combination hair shampoo-conditioner composition. The compositions disclosed by Anguillo et al. were found to clean efficiently but are inefficient in imparting conditioning properties to the hair.

Gerstein, in U.S. Patent No. 3,990,991, discloses a hair shampoo-conditioner composition comprising major amounts of an amphoteric surfactant and an ethoxylated or propoxylated cryptoanionic surfactant, with a minor amount of a cationic surfactant or cationic polymer. U.S. Patent No. 4,061,602 to Oberstar et al. discloses a conditioning shampoo composition, comprising an amphoteric surfactant, an anionic surfactant and a cationic derivative of a naturally-occurring polymer, that cleans and imparts conditioning properties to the hair. Koehler et al. in U.S. Patent No. 4,273,760 discloses a hair conditioning shampoo including a cationic polymer, an anionic surfactant and a nonionic surfactant that conditions and cleanses the hair.

Barker in U.S. Patent No. 4,247,538 discloses a conditioning shampoo comprising an amphoteric surfactant base, a cationic surfactant and an anionic macrocolloid polymer. Other hair shampoo-conditioner compositions, disclosed by Homma et al., in U.S. Patent No. 4,381,259, include a cationic polymer and an anionic phosphoric acid ester surfactant, like sodium POE(3)lauryl phosphate. Hirota et al., in U.S. Patent No. 4,479,893 discloses using a monoester or diester of phosphoric acid with an anionic, nonionic or amphoteric surfactant and a silicone conditioning compound to provide a hair shampoo-conditioner composition. Cseh, in U.S. Patent No. 4,676,978, discloses a conditioning shampoo that includes a polycationic guar derivative, a hardenable cationic polycondensation product, a nonionic surfactant, and a film-forming polymer in an anionic surfactant-based shampoo. Scandel, in U.S. Patent No. 4,832,872, teaches a hair shampoo-conditioner including an anionic surfactant, a conditioning amine oxide, and a conditioning cationic quaternary polymer. FR-A-2 548 019 discloses a cosmetic composition for the treatment of hair containing (a) at least one cationic curface-active agent which can be disbursed in water, (b) at least one water-saluble quaternized cationic polymer of the ionene type and (c) at least one cationic silicone polymere. There must be at least two cationic polymers. The cationic polymere has a moleculare weight of up to 100.000 and can be present in concentrations of up to 7%.

The following additional patents and publications also are directed to hair shampoo-conditioner compositions and compounds used in hair shampoo-conditioner compositions: A. Hunting, "The Function of Polymers in Shampoos and Conditioners", Cosmet. Toiletries, 99(6), 57-60, 1984; Coney, U.S. Patent No. 3,793,210; Olson, Jr. et al., U.S. Patent No. 3,697,452; Hewitt, U.S. Patent Nos. 3,755,559, 3,849,348 and 3,642,577; Tarasov et al., U.S. Patent No. 3,996,146; Birkofer, U.S. Patent No. 3,926,840; Barker U.S. Patent No. 3,668,136. The prior art teaching that an amphoteric detergent is necessary in a shampoo-conditioner composition is exemplified by the following patents directed to conditioning shampoos: U.S. Patent Nos. 3,313,734; 3,962,418; 2,999,069; 3,055,836; 3,996,146; 4,009,256 and 3,400,198. These patents, and others, teach the necessity of including an amphoteric or polar nonionic component in the composition to achieve compatibility between the cationic conditioning compound and the remaining components of the shampoo formulation. As will be shown in the following detailed description of the invention, these references fail, singly or in combination, to anticipate or suggest the composition and method of the present invention, wherein a polymeric cationic conditioning compound and a cationic conditioning surfactant are combined with an anionic cleansing surfactant and a fatty ester to provide a metastable hair shampoo-conditioner composition that effectively, and simultaneously, cleanses the hair and imparts improved conditioning properties to the hair. Surprisingly, the composition of the present invention is sufficiently stable to resist phase separation even though both an anionic surfactant and cationic components are present in the composition. Furthermore, the composition demonstrates a superior ability to deposit conditioning agents on the hair without exhibiting an excessive build-up of the conditioning agents on the hair shaft after repeated shampooings. Therefore, and in accordance with the present invention, the hair is cleansed and, simultaneously, excellent hair conditioning properties are imparted to the hair by a method of contacting the hair with a composition comprising an anionic cleansing surfactant, a polymeric cationic conditioning compound, a cationic conditioning surfactant and a fatty ester. Consequently, the method of the present invention both cleanses the hair and conditions the hair to provide more manageable and esthetically-pleasing hair in a single application of the shampoo-conditioner composition to the hair.

### SUMMARY OF THE INVENTION

In brief, the present invention relates to a hair shampoo-conditioner composition comprising:
(a) from 1 % to 15 % by weight of an anionic cleansing surfactant;
(b) from 0.1 % to 2 % by weight of a polymeric cationic conditioning compound having an average molecular weight of at least 100,000;
(c) from 0.2 % to 10 % by weight of a cationic conditioning surfactant;
(d) from 0.1 % to 3 % by weight of a nonionic fatty ester; and
(e) a carrier comprising water.

Under another aspect the present invention relates to a method of treating hair to simultaneously cleanse the hair and impart conditioning properties to the hair, said method comprising contacting the hair with a composition defined above; and thereafter rinsing the hair.

Preferred embodiments of the invention are recited in the dependent claims.

Optionally, an amphoteric surfactant, like a betaine or a hydroxysultaine, or a nonionic surfactant, like an alkanolamide, can be included in the composition to improve the esthetic properties and consumer appeal of the composition. Consequently, treating the hair with single application of an aqueous composition including an anionic cleansing surfactant, such as an alkyl ether sulfate, like sodium lauryl ether sulfate; a polymeric cationic conditioning compound, like a quaternized guar gum; a cationic conditioning surfactant, such as a quaternary ammonium compound including a fatty amidoalkyl substituent, like a long-chain alkamidopropyl trimonium chloride or a quaternized phosphate ester, such as tri(C₈-C₂₂ alkamidopropyl PG-dimonium chloride) phosphate; and a fatty ester, like cetearyl octanoate or isopropyl myristate, effectively cleanses the hair and simultaneously imparts excellent wet stage and excellent dry stage conditioning properties to the hair. Surprisingly and unexpectedly, hair treated with an easy-to-apply anionic surfactant-based composition of the present invention is thoroughly cleansed and exhibits improved physical and cosmetic properties, such as gloss, thickness, manageability, softness and body.

Thus the hair-treating composition of the present invention cleanses the hair and imparts improved physical properties and cosmetic properties to the hair in a single application.

The hair shampoo-conditioner composition is capable of cleansing the hair and imparting improved physical and cosmetic conditioning properties to the hair over a pH range of about 3 to about 8.

In particular, the method of treating hair comprises contacting the hair with a composition having a pH of between about 3 and 8 and including an anionic cleansing surfactant, a polymeric cationic conditioning compound, a cationic conditioning surfactant and a fatty ester, in a suitable carrier, and optionally, an amphoteric surfactant, a nonionic surfactant or a combination thereof, rinsing the hair; then drying the hair, to cleanse the hair and simultaneously impart improved physical and cosmetic conditioning properties to the hair in a single application of the composition.

The cationic conditioning surfactant may be a quaternary ammonium compound including a fatty amidoalkyl substitutent or a quaternized phosphate triester; the amphoteric surfactant may be betaine or a hydroxypropylsultone; the nonionic surfactant which may be present in a concentration of from 0% to 5% by weight may be an alkanolamide, or combinations thereof.

The polymeric cationic conditioning compound may be quaternized guar gum, the cationic conditioning surfactant may be ricinoleamidopropyl trimonium chloride or linoleamidopropyl PG-dimonium chloride phosphate, or a combination thereof, and the fatty ester may be cetearyl octanoate, ispropyl myristate or C₁₂₋₁₅ alcohols benzoate.

### BRIEF DESCRIPTION OF THE DRAWINGS

The above and other aspects and novel features of the present invention will become apparent from the following detailed description of the preferred embodiments, as illustrated in the accompanying figures showing the improved hair conditioning properties imparted by using the method and composition of the present invention, wherein:
FIG. 1 is a series of bar graphs comparing a hair shampoo-conditioner composition of the present invention to a leading commercial hair shampoo-conditioner for an ability to deposit a cationic conditioning compound on shampooed hair;
FIG. 2 is a series of bar graphs comparing the wet stage normalized combing energy of hair treated with a composition of the present invention to hair treated with PERT PLUS;
FIG. 3 is a series of bar graphs comparing the dry stage normalized combing energy of hair treated with a composition of the present invention to hair treated with PERT PLUS;
FIG. 4 is a series of bar graphs comparing foaming properties of a composition of the present invention to the foaming properties of PERT PLUS;
FIGS. 5 and 6 are series of bar graphs comparing conditioning properties imparted to hair shampooed with a hair shampoo-conditioner of the present invention to conditioning properties imparted to hair shampooed with PERT PLUS;
FIG. 7 is a series of bar graphs comparing the foaming properties of a composition of the present invention to the foaming properties of PERT PLUS;
FIG. 8 is a series of bar graphs comparing the wet stage and dry stage conditioning properties imparted to the conditioning properties imparted to hair shampooed with a composition of the present invention to the conditioning properties imparted to hair shampooed with PERT PLUS;
FIGS. 9 and 10 are series of bar graphs comparing the ability of hair shampoo-conditioner compositions of the present invention to impart wet combing and dry combing properties to a shampooed hair tress to the ability of a leading commercial hair-shampoo conditioner; and
FIGS. 11 and 12 are series of bar graphs comparing the ability of hair shampoo-conditioner compositions of the present invention to impart wet combing and dry combing properties to a shampooed hair tress to the ability of a leading commercial hair shampoo-conditioner and of a leading commercial hair conditioner to impart conditioning properties to a shampooed hair tress.

### DETAILED DESCRIPTION OF THE INVENTION

A hair shampoo-conditioner composition of the present invention comprises an anionic cleansing surfactant; a polymeric cationic conditioning compound; a cationic conditioning surfactant; and a fatty ester, in a suitable carrier. In accordance with an important feature of the present invention, the hair shampoo-conditioner composition includes an anionic cleansing surfactant and substantive cationic conditioning agents to both cleanse and condition the hair in a single application of the composition to the hair. Surprisingly and unexpectedly, the hair shampoo-conditioner composition demonstrates excellent stability in regard to resisting phase separation and in regard to resisting interaction between the anionic and cationic components, thereby avoiding the necessity of including an amphoteric surfactant in the composition. Optionally, however, an amphoteric surfactant, or a nonionic surfactant, or a combination thereof, can be included in the composition to impart improved physical properties, and therefore enhanced consumer appeal, to the composition.

The easy-to-apply composition effectively cleanses the hair and imparts excellent wet comb and dry comb conditioning properties to the hair. In general, the cleansed hair demonstrates improved physical and cosmetic conditioning properties, such as gloss, thickness, softness, manageability and body. As will be demonstrated more fully hereinafter, it is surprising and unexpected for a composition of the present invention, including an anionic cleansing compound and cationic conditioning compounds, but absent an amphoteric surfactant, both to cleanse the hair and to impart such improved conditioning properties to the hair.

The anionic cleansing surfactant used in the composition and method of the present invention includes any of the anionic surfactants known or previously used in the art of hair shampoos. However, an anionic cleansing surfactant is a necessary ingredient in the composition of the present invention because it effectively cleanses the hair and generates a high, stable, foam level that consumers equate with cleaning efficiency. Nonionic and amphoteric surfactants generally are not as effective in cleansing the hair and do not provide the high foam level desired by consumers. Therefore, nonionic and amphoteric surfactants are unsatisfactory as the primary cleansing surfactant in a composition of the present invention. However, optionally, nonionic or amphoteric surfactants can be included in a composition of the present invention to help increase and stabilize foam, to provide a suitable viscosity, or to furnish other functional or esthetic properties to the composition.

Usually, the anionic cleansing surfactant includes a hydrophobic moiety, such as a carbon chain including from about eight carbon atoms to about 30 carbon atoms, and particularly from about twelve carbon atoms to about twenty carbon atoms; and further includes a hydrophilic moiety, such as sulfate, sulfonate, carbonate, phosphate or carboxylate. Often, the hydrophobic carbon chain is etherified, such as with ethylene oxide or propylene oxide, to impart a particular physical property, such as increased water solubility or reduced surface tension, to the anionic cleansing surfactant.

The anionic cleansing surfactants are well-known and have been widely used in the art of hair shampoos. Therefore, suitable anionic cleansing surfactants include, but are not limited to, compounds in the classes known as alkyl sulfates, alkyl ether sulfates, alkyl ether sulfonates, sulfate esters of an alkylphenoxy polyoxyethylene ethanol, alpha-olefin sulfonates, beta-alkyloxy alkane sulfonates, alkyl arylsulfonates, alkyl carbonates, alkyl ether carboxylates, fatty acids, sulfosuccinates, sarcosinates, octoxynol or nonoxynol phosphates, taurates, fatty taurides, sulfated monoglycerides, fatty acid amido polyoxyethylene sulfates and isothienates; or combinations thereof. Many additional anionic cleansing surfactants are described in McCUTCHEON'S, DETERGENTS AND EMULSIFIERS, 1989 ANNUAL, published by McCutcheon Division, MC Publishing Co., and herein incorporated by reference.

Usually, the anionic cleansing surfactant is present in the composition as a neutralized salt in the form of a sodium, potassium, lithium, ammonium, alkylammonium or hydroxyalkylammonium salt, wherein the alkyl moiety includes from one to about three carbon atoms. The alkyl sulfates and alkyl ether sulfates are particularly effective classes of anionic cleansing surfactants. Consequently, exemplary anionic cleansing surfactants that are useful in the composition and method of the present invention include, but are not limited to, the ammonium, monoethanolamine, diethanolamine, triethanolamine, isopropylamine, sodium, potassium, lithium or magnesium salt of lauryl sulfate, dodecylbenzenesulfonate, lauryl sulfosuccinate, lauryl ether sulfate, lauryl ether carboxylate, lauryl sarcosinate, cocomethyl tauride, and sulfosuccinate half ester amide; or combinations thereof. An example of an especially useful anionic cleansing surfactant is a combination of a lauryl sulfate salt and a lauryl ether sulfate salt.

In accordance with an important feature of the present invention, the anionic cleansing surfactant is present in the composition in an amount ranging from about 1% to about 15% by weight of the composition. It has been found that if the anionic cleansing surfactant is present in an amount of less than about 1% by weight of the composition, then the hair is not sufficiently cleansed when contacted with a composition of the present invention. In addition, if an anionic cleansing surfactant is omitted from the composition, then the composition is unstable and separates into distinct phases in less than about 30 minutes. Furthermore, if the anionic cleansing surfactant is present in amounts greater than about 15% by weight of the composition, the anionic cleansing surfactant either may form a complex with the cationic conditioning components of the composition, thereby leading to precipitation of the complex, or may solubilize a portion of the cationic components therefore making the solubilized portion essentially unavailable for deposition on the hair shaft during shampooing.

Accordingly, it has been found that the anionic cleansing surfactant is included in the hair shampoo-conditioner composition of the present invention in a preferred amount ranging from about 3% to about 12% by weight of the composition, and to achieve the full advantage of the present invention, from about 9% to about 12% by weight of the composition. Furthermore, surprisingly and unexpectedly, even when such a low amount of anionic cleansing surfactant is included in the composition, the presence of the polymeric cationic compound and cationic surfactant does not adversely affect the generation of an acceptable and stable foam for consumer acceptance.

In accordance with another important feature of the present invention, the hair shampoo-conditioner composition includes a polymeric cationic conditioning compound that is substantive to the hair and imparts conditioning properties to the hair. It has been found that synthetic or naturally-derived polymers having a quaternized nitrogen atom are useful in the composition and method of the present invention. Such useful polymers have a weight average molecular weight of at least 100,000, and preferably of at least 200,000, and up to about 1,000,000. A preferred weight average molecular weight is from about 250,000 to about 750,000.

Synthetic quaternized polymers useful in the composition of the present invention include, but are not limited to, polyquaternium-1, polyquaternium-2, polyquaternium-5, polyquaternium-6, polyquaternium-7, polyquaternium-8, polyquaternium-9, polyquaternium-11, polyquaternium-12, polyquaternium-13, polyquaternium-14, polyquaternium-15, and mixtures thereof, wherein the compound designation is the name adopted for the compound by the Cosmetic, Toiletry and Fragrance Association, and found in the CTFA Cosmetic Ingredient Dictionary, published by the Cosmetic, Toiletry and Fragrance Association, Inc., Washington, D.C. (1982), or in the 1985 Supplement, hereinafter The CTFA Dictionary.

In particular, the naturally-derived quaternized polymers are especially useful in the composition and method of the present invention. Examples of the quaternized naturally-derived polymers include, but are not limited to, the quaternized cellulose and guar-based compounds, compounds designed in The CTFA Dictionary as polyquaternium-4, polyquaternium-10, guar hydroxypropyltrimonium chloride, and mixtures thereof. In addition, the synthetic and the naturally-derived quaternized polymers can be used in combination. An especially useful quaternized polymer is guar hydroxypropyltrimonium chloride, and sold commercially under the brandname HI-CARE 1000, by Alcolac, Linthicum, MD. Other commercially-available quaternized guar based conditioning agents include JAGUAR C-13, JAGUAR C-15 and JAGUAR C-17, from Celanese Plastics and Specialties Co., Louisville, KY. Useful quaternized cellulosic compounds include, but are not limited to, CELQUAT H60 and CELQUAT L200, from National Starch and Chemical Corp., Bridgewater, NJ and QUATRISOFT LM-200 from Amerchol Corp., Edison, NJ.

It should be understood that a cationic polymer is essential to the present invention. Compositions including a nonsubstantive, nonionic polymer, like methocel and hydroxypropylcellulose, as opposed to a quaternized guar gum, do not impart conditioning properties to treated hair, and also are unstable leading to a relatively fast phase separation. In contrast, the polymeric cationic conditioning compound included in the present composition is substantive to the hair, and also helps stabilize the composition by effectively preventing an interaction between the anionic cleansing surfactant and cationic conditioning surfactant and by providing an emulsified composition that resists phase separation under normal storage conditions. The polymeric cationic conditioning compound, present in a low amount of 2% or less by weight of the composition, does impart some conditioning properties to the shampooed hair. However, the polymeric cationic conditioning compound primarily acts to stabilize the emulsion and to reduce or eliminate interactions between the anionic cleansing surfactant and the cationic conditioning surfactant.

The polymeric cationic conditioning compound is included in the composition of the present invention in an amount ranging from about 0.1% to about 2% by weight of the composition. To achieve the full advantage of the present invention, the cationic polymeric conditioning compound is included in the hair shampoo-conditioner composition in an amount ranging from about 0.2% to about 1% by weight of the composition. It has been demonstrated that when the polymeric cationic conditioning compound is present in an amount above about 2% by weight of the composition, then a greasy feeling is imparted to shampooed hair. This greasy feeling has been attributed to an excessive build-up of conditioning compounds on the hair. This "greasy feel" is a disadvantage of the prior art compositions that include a polymeric cationic conditioning compound. Therefore, in accordance with an important feature of the present invention, the present composition includes less than 2%, and preferably less than 1%, by weight of the polymeric cationic conditioning compound. Surprisingly, it has been demonstrated that this amount of polymeric cationic conditioning compound, i.e. from about 0.1% to about 2%, and preferably from about 0.2% to about 1%, by weight, is sufficiently low to provide clean, conditioned hair that is not greasy to the feel; and is sufficiently high to provide a stable emulsified composition and to impart conditioning properties to the hair. To achieve the full advantage of the present invention, the polymeric cationic conditioning agent is included in the composition of the present invention in an amount ranging from about 0.2% to about 1.0% by weight.

In addition to the anionic cleansing surfactant and the polymeric cationic conditioning compound, the hair shampoo-conditioner composition also includes a cationic conditioning surfactant in an amount ranging from about 0.2% to about 10%, and preferably from about 0.5% to about 5%, by weight of the composition to impart conditioning properties to the shampooed hair. In general, cationic surfactants are incompatible with anionic surfactants. However, it has been found that cationic surfactants included in a composition manufactured by the method of the present invention essentially do not interact with the anionic cleansing surfactant present in the composition. Therefore, the anionic cleansing surfactant is available to cleanse the hair and the cationic conditioning surfactant is available to condition the hair.

In particular, introducing a cationic surfactant into the composition provides: 1) an excellent hair conditioner for treating hair, and 2) surprisingly does not destabilize the composition to such a degree that an interaction between the anionic cleansing surfactant and the cationic conditioning surfactant occurs. Therefore, neither ingredient precipitation nor decreased product performance is observed. However, it has been theorized that introducing the cationic conditioning surfactant into the composition does destabilize the composition to a slight, but desirable, degree. Accordingly, the cationic conditioning surfactant is not completely soluble in the metastable composition, but is available for deposition onto hair upon hair contact, and resists removal from the hair during rinsing of the anionic cleansing surfactant from the hair. Therefore, improved and more durable conditioning properties, such as body and manageability, are imparted to the treated hair.

The most commonly-used cationic conditioning surfactant is a quaternary ammonium compound including at least one quaternized nitrogen atom. However, it has been found that the most common quaternary ammonium compounds having one, two or three long-chain, saturated alkyl groups including from about 8 to about 22 carbon atoms as substituents on the quaternary nitrogen atom, with the remaining substituents on the quaternary nitrogen atom selected from hydrogen, benzyl, short chain alkyl groups and short chain hydroxyalkyl groups, wherein the short chain alkyl and hydroxyalkyl groups include up to about four carbon atoms, are not useful in the composition of the present invention.

Quaternary ammonium compounds useful in the present invention include a long carbon chain substituent having a carbonyl moiety, like an amide moiety, or include a phosphate ester moiety. These particular quaternary ammonium compounds demonstrate exceptional compatibility with the anionic cleansing surfactant when incorporated into a hair shampoo-conditioner composition of the present invention. It has been theorized, but is not relied upon herein, that these particular quaternary ammonium compounds are more hydrophilic than quaternary ammonium compounds including long-chain, saturated alkyl groups. Accordingly, these particular hydrophilic quaternary ammonium compounds provide a composition having the desired degree of stability to resist phase separation and yet are available to deposit on, and resist rinsing from, the hair.

One of the classes of cationic surfactants found especially useful in the composition of the present invention are quaternary ammonium compounds depicted by general structural formula (I): 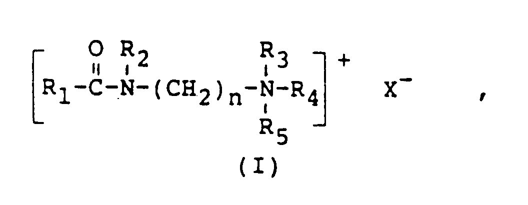
wherein R₁ is a substituted or unsubstituted, saturated or unsaturated, alkyl group including from about 5 to about 21 carbon atoms; R₂ is hydrogen or methyl; R₃, R₄ and R₅, independently, are methyl, ethyl, hydroxyethyl or benzyl; n is a numeral from one to about 10; and X is an anion selected from the group consisting of chloride, bromide, ethosulfate, methosulfate, acetate, nitrate, tosylate, phosphate and combinations thereof. A quaternary ammonium compound of general structural formula (I) demonstrates both sufficient compatibility with the anionic cleansing surfactant and a sufficient ability to impart conditioning properties to shampooed hair. In addition, the cationic conditioning surfactant of general structural formula (I) can further act as an emulsifier for water-insoluble compounds included in the composition.

Examples of useful cationic surfactants having the general structural formula (I) include, but are not limited to, compounds designated in the CTFA Dictionary as ricinoleamidopropyl trimonium chloride, ricinoleamido trimonium ethyl sulfate, hydroxy stearamidopropyl trimonium methyl sulfate and hydroxy stearamidopropyl trimonium chloride, or combinations thereof. These cationic conditioning surfactants are available commercially from CasChem Inc., Bayonne, N.J. under the brandnames SURFACTOL Q1, SURFACTOL Q4, SURFACTOL Q3 and SURFACTOL Q2, respectively. Another useful cationic surfactant having the general structural formula (I) is designated in the CTFA Dictionary as ricinoleamidopropyl ethyldimonium ethosulfate, available commercially as LIPOQUAT R from Lipo Chemicals, Inc., Paterson, NJ. In particular, these quaternary ammonium surfactants possess either a hydroxy substituent on the R₁ alkyl group of the compound depicted in general structural formula (I) and/or unsaturation in the carbon chain of the R₁ alkyl group of the compound of structural formula (I). Examples of other useful quaternary ammonium surfactants include, but are not limited to, isostearamidopropyl ethyldimonium ethosulfate, Quaternium-22 and Quaternium-26, or combinations thereof, as designated in the CTFA Dictionary. In general, however, any quaternary ammonium compound including a fatty amidoalkyl substituent can be included in the composition of the present invention as long as the resistance to phase separation and precipitation of ingredients, the cleansing efficiency and the conditioning efficiency of the composition are not adversely affected.

Another particularly useful class of quaternary ammonium compounds that can be included in the composition of the present invention are the quaternized phosphate esters, as depicted in general structural formula (II): 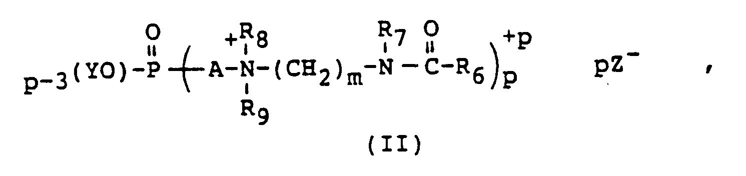
wherein R₆ is an aryl, an alkaryl, a saturated or unsaturated alkyl group, or a saturated or unsaturated hydroxylalkyl group wherein the alkyl or hydroxyalkyl group includes from about seven to about 21 carbon atoms; R₇ is hydrogen, or an alkyl or a hydroxyalkyl group including from one to about six carbon atoms; R₈ and R₉, independently, are an alkyl or a hydroxyalkyl group including from one to about six carbon atoms; A is a residue of a glycol or a triol having from two to about four carbon atoms, such as the residue of propylene glycol (-OCH₂CH(OH)CH₂-); Z is an anion selected from the group consisting of chloride, bromide, methosulfate, ethosulfate and combinations thereof; m is a numeral from one to about 10; Y is selected from the group consisting of hydrogen, an alkyl group, a hydroxyalkyl group and an aryl group, either substituted or unsubstituted, and wherein the alkyl or the hydroxyalkyl group includes from one to about 22 carbon atoms; and p is a number from 1 to 3. To achieve the full advantage of the present invention, the quaternized phosphate ester is a quaternized phosphate triester that includes the alkyl moiety of an essential fatty acid, like linoleic acid, arachidonic acid or ricinoleic acid, as the R₆ substituent of the compound. For example, the quaternized phosphate ester of general structural formula (II) that includes the alkyl moiety of an essential fatty acid as the R₆ substituent and wherein the number p is 3.

The essential fatty acid substituent helps the compound impart conditioning properties to the hair and also provides skin conditioning properties to the scalp. An example of an especially useful quaternized phosphate triester is depicted in structural formula (III), available commercially under the brandname PHOSPHOLIPID EFA, from Mona Industries, Paterson, NJ, and having the proposed CTFA Dictionary designation as 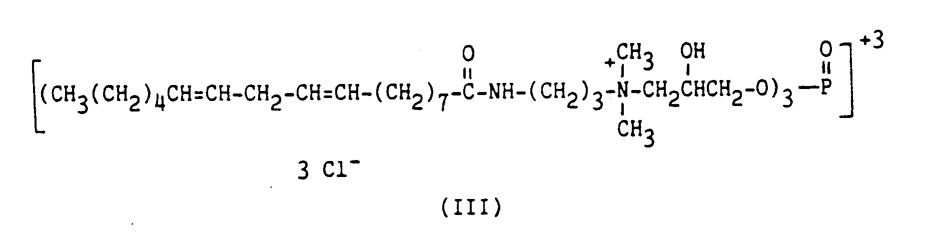
linoleamidopropyl PG-dimonium chloride phosphate. This particular compound has p equal to 3 and includes the alkyl moiety of linoleic acid as the substituent R₆. It should be understood that the monophosphate ester (i.e. p=1) and diphosphate ester (i.e. p=2) of the quaternized phosphate ester illustrated in general structural formula (II) also can be used in the composition of the present invention as long as the basic properties of the hair shampoo-conditioner are not adversely affected. For example, suitable monophosphate and diphosphate esters of general structural formula (II) include Y as hydrogen, if the composition pH is sufficiently low such that the acid form of the phosphoric acid ester is present, as opposed to the neutralized, salt form; or Y as an alkyl group, a hydroxyalkyl group or an aryl group.

In addition to the anionic cleansing surfactant, the cationic polymeric conditioning compound and the cationic conditioning surfactant, the hair shampoo-conditioner composition also includes a fatty ester. The fatty ester serves both as a conditioning agent and as a secondary emulsifier. In addition, it has been theorized, but is not relied upon herein, that the nonionic nature of the fatty ester compound also may assist in stabilizing the composition by forming a complex or a micelle with the cationic conditioning surfactant, thereby helping prevent an interaction between the anionic and the cationic components in the composition.

The fatty ester is included in the composition in an amount ranging from about 0.1% to about 3% by weight of the composition. Preferably, the fatty ester is present in an amount ranging from about 0.5% to about 2% by weight of the composition. The fatty component of the fatty ester can be derived from a fatty acid or a fatty alcohol, or a combination thereof. In addition, the fatty ester can be a straight chain fatty ester, like isopropyl myristate; a branched chain fatty ester, like Purcellin Oil; a benzoate ester, like C₁₂₋₁₅ alcohols benzoate; or a combination thereof.

For example, a useful class of fatty esters are derived from carboxylic acids having from about six to about 12 carbon atoms, including both branched and straight chain carboxylic acids. In general, the C₆ to C₁₂ carboxylic acid is esterified with a fatty alcohol including from about 12 to about 22 carbon atoms to provide a fatty (C₁₂ to C₂₂) ester of a C₆ to C₁₂ carboxylic acid that is useful in the present invention. Such fatty alcohols include, but are not limited to, lauryl alcohol, myristyl alcohol, cetyl alcohol, cetearyl alcohol, stearyl alcohol, isostearyl alcohol, oleyl alcohol, tallow alcohol, behenyl alcohol and mixtures thereof. Accordingly, fatty (C₁₂ to C₂₂) esters of C₆ to C₁₂ carboxylic acids useful in the composition and method of the present invention include, but are not limited to, cetyl octanoate, stearyl heptanoate, stearyl caprylate, stearyl octanoate, lauryl octanoate, myristyl heptanoate, and oleyl octanoate, or mixtures thereof. These fatty esters can occur naturally or can be synthesized. A particularly useful fatty (C₁₂ to C₂₂) ester of a branched C₆ to C₁₂ carboxylic acid is designed in the CTFA Dictionary as cetearyl octanoate, a branched fatty acid ester available commercially under the brandname PURCELLIN OIL, from Dragoco, Inc., Totowa, N.J.

In place of, or in combination with, the fatty (C₁₂ to C₂₂) ester of a C₆ to C₁₂ carboxylic acid, a fatty ester derived from a fatty acid including from about eight to about 22 carbon atoms esterified with an alcohol including from one to about six carbon atoms can be included in the composition of the present invention. Examples of such fatty esters include, but are not limited to, isopropyl myristate, isopropyl palmitate, isopropyl laurate, isopropyl linoleate, isopropyl isostearate, isopropyl oleate, isopropyl stearate, isopropyl tallowate, isopropyl ricinoleate, methyl laurate, methyl linoleate, methyl myristate, methyl stearate, methyl ricinoleate, methyl caprylate, methyl oleate, methyl palmitate, methyl stearate, methyl behenate, methyl soyate, methyl tallowate, isopropyl behenate, isopropyl soyate, propyl oleate, butyl oleate, butyl stearate, methyl coconate, methyl lardate, isobutyl palmitate, butyl myristate, ethyl palmitate, ethyl myristate, ethyl oleate, ethyl stearate, isobutyl stearate, isobutyl myristate and combinations thereof.

Another class of fatty esters that can be included in the composition of the present invention, either alone or in combination with the fatty esters described above, is the benzoate esters. Suitable benzoate esters include esters of benzoic acid wherein the esterifying alcohol includes from about eight carbon atoms to about 22 carbon atoms. Examples of suitable benzoate esters include, but are not limited to, the commercial products FINSOLV TN, benzoic acid esterified with fatty alcohols including from about 12 to about 15 carbon atoms; FINSOLV SB, isostearyl benzoate; FINSOLV P, PPG-15 stearyl ether benzoate; or combinations thereof, all available from Finetex Inc., Elmwood Park, NJ.

In addition to the above-described essential ingredients, other common cosmetic components and additives can be included in the composition of the present invention, as long as the basic properties of the hair shampoo-conditioner composition are not adversely affected. Such optional cosmetic components and additives include, but are not limited to, nonionic surfactants, amphoteric surfactants, fragrances, dyes, hair colorants, opacifiers, pearlescing agents, thickeners, dandruff control agents, hydrotropes, foam stabilizers, solubilizers, preservatives, water softening agents, acids, alkalis, buffers and the like. These optional components and additives usually are present in weight percentages of from 0% to less than about 5% by weight each, and usually from about 0.1% to about 20% by weight of the composition in total.

For example, to improve consumer acceptance, both skin mildness and enhanced composition esthetics can be achieved by optionally including an amphoteric surfactant in the hair shampoo-conditioner in an amount ranging from 0% to about 5% by weight of the composition. It has been found that if an amphoteric surfactant is included in the composition, the total amount of anionic cleansing surfactant and optional amphoteric surfactant in the composition should not exceed approximately 20% by weight. At amounts of anionic surfactant above about 15% by weight, or at a combined amount of anionic surfactant and amphoteric surfactant above about 20% by weight of the composition, composition performance decreases because either: a) the anionic surfactant is present in a sufficiently large amount to interact with the cationic components of the composition causing either precipitation of composition ingredients or reduced composition performance, or b) the anionic surfactant and amphoteric surfactant are present in a sufficiently large amount to solubilize the cationic components, and therefore reduce the amount of cationic components that deposit on the hair. As will be demonstrated more fully hereinafter, it also has been shown that if an anionic cleansing surfactant is omitted from the composition and an amphoteric surfactant is included, then the composition is sufficiently unstable such that phase separation occurs within a short time.

Suitable amphoteric surfactants that can be included in the present invention include, but are not limited to, betaines, hydroxypropylsultaines and amine oxides, or combinations thereof. Examples of amphoteric surfactants include, but are not limited to, cocamidopropyl betaine, lauramidopropyl betaine, coco/oleamidopropyl betaine, coco betaine, oleyl betaine, cocamidopropyl hydroxysultaine, tallowamidopropyl hydroxysultaine and dihydroxyethyl tallow glycinate, or combinations thereof. A particularly useful amphoteric surfactant is cocamidopropyl betaine, sold commercially under the brandnames TEGO-BETAINE L10 and TEGO-BETAINE L7, by Goldschmidt Chemical Corp., Hopewell, VA. In general, however, any amphoteric surfactant can be included in the composition of the present invention as long as the stability, the conditioning and the cleansing efficiency of the composition are not adversely affected.

The hair shampoo-conditioner compositions of the present invention also can include nonionic surfactants to help impart esthetic, physical or cleansing properties to the composition. Likewise, the compositions can include other emulsifiers, conditioning agents, inorganic salts, humectants and similar materials to provide the composition with desirable esthetic or physical properties. Generally, such optional ingredients are present in weight percentages of from 0% to about 5% each, and from 0% to about 20% in total, relative to the total weight of the composition.

For example, representative nonionic surfactants that can be included in the hair shampoo-conditioner composition of the present invention include esters of polyols and sugars; fatty acid alkanolamides; polyethylene glycols; the ethoxylated or propoxylated alkylphenols; ethoxylated or propoxylated fatty alcohols; and the condensation products of ethylene oxide with long chain amides. These nonionic surfactants, as well as numerous others not cited herein, are well known in the art and are fully described in the literature, such as McCUTCHEON'S, DETERGENTS AND EMULSIFIERS, 1989 Annual, published by McCutcheon Division, MC Publishing Co.

In particular, a nonionic alkanolamide can be included in the composition to provide composition thickening and foam stability. The alkanolamide can be included in an amount ranging from 0% to about 5% by weight of the composition. The alkanolamides are preferred thickeners because the usual organic thickeners used in cosmetics, such as sodium alginate; guar gum; xanthan gum; gum arabic; cellulose derivatives such as methylcellulose, hydroxybutylcellulose, hydroxyethylcellulose, hydroxypropylcellulose and carboxymethylcellulose; and various synthetic polymeric thickeners, such as the polyacrylic acid derivatives, are incompatible with the essential ingredients of the composition. Accordingly, suitable alkanolamides include, but are not limited to, those known in the art of hair care formulations, such as cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA and combinations thereof.

The composition also can include optional conditioning agents and emulsifiers, like fatty alcohols, such as myristyl alcohol, lauryl alcohol, stearyl alcohol, cetearyl alcohol or cetyl alcohol. In general, the fatty alcohol conditioning agents, and other conditioning agents well-known to those skilled in the art optionally can be included in the present hair shampoo-conditioner composition in an amount ranging from 0% to about 3% by weight of the composition.

The carrier of the hair shampoo-conditioner composition of the present invention is predominantly water, but nonaqueous solvents also can be included to help solubilize composition ingredients that are not sufficiently soluble in water, to adjust the viscosity of the composition or to act as a humectant. Suitable solvents include polyols, like glycerol; glycols, like ethylene glycol, propylene glycol and hexylene glycol; or mixtures thereof. The optional nonaqueous solvents should not adversely affect the ability of the composition to cleanse and condition the hair or adversely affect consumer appeal of the composition. A nonaqueous solvent can be present in the hair shampoo-conditioner composition of the present invention in an amount ranging from 0% to about 5% by weight of the composition.

To achieve the full advantage of the present invention, the hair shampoo-conditioner composition is a relatively viscous mixture that is stable indefinitely at temperatures normally found in commercial product storage and shipping. A composition of the present invention generally is an emulsion that is stable and that resists phase separation or precipitation of composition ingredients at a temperature of about 20°C to about 25°C essentially indefinitely. The compositions also have demonstrated sufficient stability to phase separation or precipitation of ingredients at temperatures normally found in commercial product storage and shipping to remain unaffected for periods of one year or more.

A sufficiently viscous hair shampoo-conditioner composition results from a judicious selection of the anionic cleansing surfactant, the polymeric cationic conditioning compound, the cationic surfactant and the fatty ester. As will be demonstrated more fully hereinafter, it has been found that when manufactured according to the preferred method, a stable, viscous composition having enhanced consumer appeal results. Although other methods of manufacture provide useful compositions, it has been found that the preferred method of manufacture provides metastable compositions exhibiting both a sufficiently long storage stability and an enhanced ability to impart conditioning properties to shampooed hair.

In accordance with the method of the present invention, several hair shampoo-conditioner compositions were prepared, then applied to hair, to demonstrate the ability of a single application a composition, comprising an anionic cleansing surfactant; a polymeric cationic conditioning compound; a cationic conditioning surfactant; and a fatty ester, to simultaneously cleanse the hair and impart hair-conditioning properties to the hair. It has been demonstrated that a metastable hair shampoo-conditioner composition of the present invention maximizes the hair conditioning properties imparted to the hair. Although the mechanism of interaction between the essential ingredients that provides a relatively stable composition and allows a maximum deposition of conditioning compounds on the hair is not known precisely, it has been theorized that complexes formed during the manufacture of the composition effectively isolate the cationic quaternary ammonium functionalities from contact with the anionic cleansing surfactants. Consequently, because contact between the anionic and cationic components of the composition is effectively prevented, the cationic components are not precipitated from the composition, do not otherwise interact with the anionic surfactant leading to decreased effectiveness, and are therefore available to effectively deposit onto, and condition, the hair shaft. Similarly, the anionic cleansing surfactant also is available to effectively cleanse the hair. Furthermore, and as will be demonstrated more fully hereinafter, salon tests have demonstrated that a stable and sufficiently high foam level is generated during shampooing, thereby providing enhanced consumer appeal, even at the relatively low amounts of anionic cleansing surfactant present in the composition; and that excellent conditioning properties are imparted to the hair.

To demonstrate the new and unexpected results provided by the hair shampoo-conditioner of the present invention, the following Examples 1 through 28 were prepared. The preferred method of manufacturing the hair shampoo-conditioner compositions will be discussed in detail hereinafter. The compositions of Examples 1-9 illustrate the storage stability of the shampoo-conditioner compositions; and the cleansing efficiency and conditioning properties imparted by a composition of the present invention. The weight percentage listed in each of the following examples represent the actual amount of each ingredient present in the hair shampoo-conditioner composition.

| INGREDIENT (% by weight) | EX. 1 | EX. 2 |
|---|---|---|
| Quaternized Guar Gum ¹⁾ | 1.50 | 1.50 |
| Ricinoleamidopropyl Trimonium Chloride ²⁾ | 1.65 | 1.65 |
| Cocamide MEA | - | 1.50 |
| Linoleamidopropyl PG-Dimonium Chloride Phosphate ³⁾ | 0.60 | 0.60 |
| Ammonium Lauryl Sulfate | 6.14 | - |
| Ammonium Lauryl Ether Sulfate (1 mole ethylene oxide) | 6.14 | - |
| Cocamidopropyl Betaine | - | 11.46 |
| Cetearyl Octanoate ⁴⁾ | 2.00 | 2.00 |
| Deionized Water | q.s. | q.s. |

| | | |
|---|---|---|
| 1) Polymeric cationic conditioning compound; Guar hydroxypropyltrimonium chloride, HI-CARE 1000, from Alcolac, Linthicum, MD., (100% active). | | |
| 2) Cationic conditioning surfactant; SURFACTOL Q1, from CasChem, Bayonne, NJ., (55% active in propylene glycol). | | |
| 3) Cationic conditioning surfactant; PHOSPHOLIPID EFA, from Mona Industries, Paterson, NJ., (30% active). | | |
| 4) Fatty ester; PURCELLIN OIL, from Dragoco, Inc., Totowa, NJ., (100% active). | | |

The composition of EX. 1 is a composition of the present invention including only the essential ingredients and absent any optional ingredients such as amphoteric or nonionic surfactants. The composition of EX. 1 was an opacified liquid having a relatively low viscosity; demonstrated excellent storage stability; and exhibited no phase separation or ingredient precipitation after storage at about 25°C for an extended time. In contrast, the composition of EX. 2 does not include the essential anionic cleansing surfactant, but does include an optional amphoteric surfactant and an optional nonionic surfactant. The composition of EX. 2 separated into a white, curdly layer and a clear layer about 20 minutes after preparation. Accordingly, it was demonstrated that the anionic cleansing surfactant imparts stability to the composition, and therefore is an essential ingredient. As will be demonstrated more fully hereinafter, compositions of the present invention including both an essential anionic surfactant and an optional amphoteric or nonionic surfactant also demonstrated excellent composition stability.

| INGREDIENT (% by weight) | EX. 3 | EX. 4 | EX. 5 | EX. 6 | EX. 7 | EX. 8 |
|---|---|---|---|---|---|---|
| Quaternized Guar Gum ¹⁾ | 1.00 | 0.75 | 1.00 | 1.00 | 1.50 | 1.50 |
| Ricinoleamidopropyl Trimonium Chloride ²⁾ | 1.38 | 1.65 | 1.65 | 1.65 | 1.65 | 1.65 |
| Cocamide MEA | 2.00 | - | - | 1.00 | 1.50 | - |
| Linoleamide DEA | - | 2.00 | 2.00 | - | - | - |
| Linoleamidopropyl PG-Dimonium Chloride Phosphate ³⁾ | 0.45 | 0.30 | 0.60 | 0.60 | 0.60 | 0.60 |
| Ammonium Lauryl Sulfate | 4.95 | 10.20 | 6.00 | 4.50 | 6.10 | 4.00 |
| Ammonium Lauryl Ether Sulfate (1 mole ethylene oxide) | 4.95 | - | 4.20 | 4.50 | 6.10 | 4.00 |
| Cocamidopropyl Betaine | 4.05 | 3.00 | 4.20 | 3.00 | - | 4.00 |
| Cetearyl Octanoate ⁴⁾ | 2.50 | 1.00 | 1.00 | 2.00 | 2.00 | 2.00 |
| Deionized Water | q.s. | q.s. | q.s. | q.s. | q.s. | q.s. |
| Optional Ingredients (dye, fragrance, preservatives, etc.) | q.s. to 100% | q.s. to 100% | q.s. to 100% | q.s. to 100% | q.s. to 100% | q.s. to 100% |

The compositions of EXS. 3-8 were opaque to pearlescent liquids having a relatively low viscosity. The compositions demonstrated excellent storage stability, exhibiting no phase separation or ingredient precipitation after storage at about 25°C for about 1 year. The compositions of EXS. 3-8, when applied to a bleached, waved tress of human hair, demonstrated excellent foaming properties and imparted excellent detangling, wet combing and dry combing properties to the hair tress. The compositions of EXS. 3-6 included each of the essential ingredients, and in addition, included an optional nonionic alkanolamide and an optional amphoteric surfactant. The composition of EX. 7 included a nonionic alkanolamide, but lacked an amphoteric surfactant. The composition of EX. 8 included an amphoteric surfactant, but lacked a nonionic alkanolamide. It was found that each of the compositions of EXS. 3-8 imparted good wet combing properties to the hair. It also was found that the particular type of optional alkanolamide, such as a monoethanolamide or a diethanolamide, essentially did not affect either the stability or the performance of the composition. However, the monoethanolamide-containing compositions were esthetically more pleasing, and therefore demonstrated enhanced consumer appeal. Comparing the composition of EX. 4 to the compositions of EX. 3 and EXS. 5 through 8 showed that including either a combination of anionic cleansing surfactants or a single anionic cleansing surfactant (EX. 4) provides a composition demonstrating sufficient stability, sufficient cleansing ability and the ability to impart hair conditioning properties to the hair.

Other hair shampoo-conditioner compositions of the present invention also were prepared by varying the identity of ingredients included in the compositions of EXS. 3-8. In particular, it was found that other anionic cleansing surfactants, such as an alpha-olefin sulfonate or a sarcosinate, can be included in the hair shampoo-conditioner composition essentially without adversely affecting the physical properties, esthetic properties or the performance properties of the composition. Likewise, an optional alkamidopropyl hydroxysultaine was substituted for the optional betaine and the physical, esthetic and performance properties were not adversely affected. As stated previously, varying the identity of the optional alkanolamide also had essentially no effect on either the physical or the performance properties of the composition.

Other hair shampoo-conditioner compositions of the present invention also were prepared by varying the identity of ingredients included in the compositions of EXS. 3-8. In particular, it was found that other anionic cleansing surfactants, such as an alpha-olefin sulfonate or a sarcosinate, can be included in the hair shampoo-conditioner compositions essentially without adversely affecting the physical properties, esthetic properties or the performance properties of the composition. Likewise, an optional alkamidopropyl hydroxysultaine was substituted for the optional betaine and the physical, esthetic and performance properties were not adversely affected. As stated previously, varying the identity of the optional alkanolamide also had essentially no effect on either the physical or the performance properties of the composition.

However, it was found that a composition absent a cationic conditioning surfactant, like the quaternized phosphate ester linoleamidopropyl PG-dimonium chloride phosphate, demonstrated a substantially decreased stability. Furthermore, it was found that the composition of EX. 6 demonstrated the best overall shelf stability, cleansing properties and ability to impart conditioning properties of the several hair shampoo-conditioner compositions that were prepared. As further observed, the composition of EX. 6 includes only about 12% by weight total of the anionic cleansing surfactants and the optional amphoteric surfactants, yet sufficiently cleans the hair and imparts excellent hair conditioning properties to the hair.

To demonstrate that the amount of optional nonionic surfactant and optional amphoteric surfactant can be varied without adversely affecting the ability of the composition to clean and condition hair, the following composition of Example 9 was prepared:

### EXAMPLE 9

| INGREDIENT | (% active by weight) |
|---|---|
| Quaternized Guar Gum ¹⁾ | 1.00 |
| Ricinoleamidopropyl Trimonium Chloride ²⁾ | 1.65 |
| Cocamide MEA | 1.00 |
| Lauramide DEA | 1.00 |
| Linoleamidopropyl PG-Dimonium Chloride Phosphate ³⁾ | 0.60 |
| Ammonium Lauryl Sulfate | 4.00 |
| Ammonium Lauryl Ether Sulfate (1 mole ethylene oxide) | 7.00 |
| Cocamidopropyl Betaine | 3.50 |
| Cetearyl Octanoate | 2.00 |
| Deionized Water | q.s. |
| Optional Ingredients (dye, fragrance, preservatives, etc.) | q.s. |

The composition of EX. 9 is similar to the composition of EX. 6, except the total amount of optional nonionic surfactant and optional amphoteric surfactant in the composition of EX. 9 exceeds the amounts of optional ingredients in EX. 6. A test comparing the composition of EX. 9 to the composition of EX. 6 demonstrated only a slightly decreased ability of the composition of EX. 9 to impart conditioning properties to treated hair. It has been theorized that the decrease in conditioning properties imparted to the shampooed hair may be attributed to rinsing a greater amount of the cationic conditioning components of the composition from the treated hair because of the greater total amount of surfactant present in the composition of EX. 9.

To demonstrate that the method of manufacturing the hair shampoo-conditioner composition of the present invention is related to the shelf stability of the composition and to the ability of the composition to impart conditioning properties to the hair, the following test was performed. First, the composition of EX. 10 was prepared. This composition includes the essential polymeric cationic conditioning compound and the essential anionic cleansing surfactant, but lacks the essential cationic conditioning surfactant and the essential fatty ester. The composition was clear and had a high viscosity. However, hair shampooed with the composition of EX. 10 demonstrated very poor wet combing properties.

### EXAMPLE 10

| INGREDIENT | (% active by weight) |
|---|---|
| Quaternized Guar Gum ¹⁾ | 1.00 |
| Cocamide MEA | 1.00 |
| Ammonium Lauryl Sulfate | 4.50 |
| Ammonium Lauryl Ether Sulfate (1 mole ethylene oxide) | 4.50 |
| Cocamidopropyl Betaine | 3.00 |
| Deionized Water | q.s. |

Then, to about 25 g (grams) of the composition of EX. 10 was added about 0.40 g of the cationic conditioning surfactant, ricinoleamidopropyl trimonium chloride (SURFACTOL Q1). Hair shampooed with the resulting composition (EX. 10A) also exhibited very poor wet combing properties. Similarly, adding about 0.15 g of the cationic conditioning surfactant linoleamidopropyl PG-dimonium chloride phosphate (PHOSPHOLIPID EFA) to 26.54 g of the composition of EX. 10, provided a composition (EX. 10B) that was jelly-like. Hair shampooed with the composition of EX. 10B demonstrated very poor wet combing properties. Likewise, adding the fatty ester cetearyl octanoate (PURCELLIN OIL) to the composition of EX. 10 provided a jelly-like composition (EX. 10C), and hair shampooed with the composition of EX. 10C demonstrated poor wet combing properties. Finally, when each of the above three ingredients, namely SURFACTOL Q1, PHOSPHOLIPID EFA and PURCELLIN OIL, were added to the composition of EX. 10, the resulting composition (EX. 10D) exhibited a viscosity of about 5000 cps (centipoises) and moderate wetting properties. The composition of EX. 10D included all of the essential ingredients of the present invention and in essentially the same amounts as the composition of EX. 6. The composition of EX. 10D outperformed the composition of EX. 10, and the compositions of EXS. 10A, 10B and 10C, in regard to imparting conditioning properties to the hair. However, the composition of EX. 6 exhibited a greater ability to cleanse and to condition the hair then the composition of EX. 10D. Accordingly, it was found that the method of manufacturing a composition of the present invention is related to the cleansing ability of the composition and to the conditioning properties imparted to hair shampooed with the hair shampoo-conditioner composition.

It should be understood that a composition of the present invention can be manufactured by a variety of methods and, regardless of the manufacturing method, the resulting composition demonstrates the advantages of shelf stability, efficient cleansing and the ability to impart conditioning properties to the hair. However, it also has been found that a particular method of manufacturing the hair shampoo-conditioner composition provides a composition that demonstrates an even greater ability to cleanse the hair and to impart conditioning properties to the shampooed hair. Therefore, to achieve the full advantage of the present invention, a hair shampoo-conditioner composition of the present invention is manufactured by first admixing the polymeric cationic conditioning compound and, if present, the optional nonionic surfactant, like an alkanolamide, with water having a temperature of from about 25°C to about 75°C, and preferably from about 40°C to about 60°C, then stirring the mixture until a homogeneous mixture results. Then, either sequentially in any order, or as a premix, the cationic surfactant, the fatty ester and, if present, the optional amphoteric surfactant are added to the homogeneous mixture. After sufficiently stirring to again form a homogeneous mixture, stirring is stopped. Then with or without stirring, the anionic cleansing surfactant is added to the homogeneous mixture. After all of the anionic surfactant is added to the homogeneous mixture, stirring is continued or resumed, until the composition cools to ambient temperature. A composition of the present invention manufactured by the above preferred method demonstrated excellent shelf stability; exhibited an excellent ability to cleanse the hair; and imparted excellent conditioning properties to shampooed hair.

To further demonstrate the ability of a composition of the present invention to cleanse and condition hair, the hair shampoo-conditioner compositions of Examples 11 through 14 were prepared. In each example, the amount of quaternized guar gum was limited to 2% or less by weight of the composition. At amounts greater than 2% by wt., the quaternized guar gum provided a composition that had a slimy, or slippery feel, and that led to an excessive build-up of quaternized guar gum, and a greasy feel, on the hair. Furthermore, at amounts greater than 2% by weight, no additional benefits, such as further increased emulsion stabilization, were observed.

| INGREDIENT (% by weight) | EX. 11 | EX. 12 | EX. 13 | EX. 14 |
|---|---|---|---|---|
| Quaternized Guar Gum ¹⁾ | 1.50 | 2.00 | 1.00 | 1.00 |
| Ricinoleamidopropyl Trimonium Chloride ²⁾ | 1.65 | 2.37 | 1.73 | 1.93 |
| Cocamide MEA | 1.50 | 1.50 | 1.00 | 1.00 |
| Linoleamidopropyl PG-Dimonium Chloride Phosphate ³⁾ | 0.60 | 0.60 | 0.60 | 0.60 |
| Ammonium Lauryl Sulfate | 4.00 | 4.00 | 4.50 | 4.50 |
| Ammonium Lauryl Ether Sulfate (1 mole ethylene oxide) | 4.00 | 4.00 | 4.50 | 4.50 |
| Cocamidopropyl Betaine | 4.00 | 4.00 | 3.15 | 3.00 |
| Cetearyl Octanoate | 2.00 | 2.00 | 1.30 | 2.00 |
| Preservative ⁵⁾ | 0.155 | 0.155 | 0.155 | 0.155 |
| Fragrance | 0.30 | 0.30 | 0.30 | 0.30 |
| Dye | q.s. | q.s. | q.s. | q.s. |
| Deionized Water | q.s. to 100% | q.s. to 100% | q.s. to 100% | q.s. to 100% |

| | | | | |
|---|---|---|---|---|
| 5) Preservative includes 0.005% BHA (butylated hydroxyanisole); 0.10% GLYDANT (DMDM hydantoin); and 0.05% KATHON CG, available from Rohm and Haas Co., Philadelphia, PA. (methylchloroisothiazolinone and methylisothiazolinone). | | | | |

The compositions of EXS. 11 through 14 were prepared by adding the quaternized guar gum to the stirred deionized water. The mixture was heated to about 50°C., then the cocamide MEA was added. The resulting mixture was heated and stirred until a homogeneous mixture was formed. Then the cocamidopropyl betaine and linoleamidopropyl PG-dimonium chloride phosphate were added, and stirring was continued until the mixture again was homogeneous. Then, after adding the ricinoleamidopropyl trimonium chloride with stirring, heating of the resulting homogeneous mixture was stopped. The ammonium lauryl ether sulfate then was added to the mixture without stirring. After completing the addition of the ammonium lauryl ether sulfate, the mixture again was stirred until homogeneous. Stirring then was stopped again, and stirring was not resumed until the entire amount of the ammonium lauryl sulfate was added to the mixture. Then, after stirring was resumed, the cetearyl octanoate, BHA, GLYDANT and KATHON CG were added to the mixture. Finally, the dye and fragrance were added to the mixture, and the resulting mixture was stirred until homogeneous. Each composition of EXS. 11 through 14 was an opaque liquid, having a viscosity in the range of from about 1000 to about 10,000 cps (centipoise), and demonstrated excellent storage stability.

The composition of Example 11 was compared to a leading present-day commercial hair shampoo-conditioner, PERT PLUS, available from Proctor and Gamble Co., Cincinnati, OH, to determine the relative ability of a composition of the present invention to effectively cleanse the hair and to simultaneously impart hair conditioning properties to the hair during shampooing. The comparative test between the composition of EX. 11 and PERT PLUS demonstrated that the composition of EX. 11 performed essentially equally to PERT PLUS, a hair shampoo-conditioner composition recognized in the industry as an effective shampoo having the ability to impart exceptional hair conditioning properties to hair.

In particular, to show that a composition of the present invention effectively cleanses the hair and imparts superior hair conditioning properties to hair, the composition of EX. 11 was compared to the commercially-available PERT PLUS shampoo-conditioner in a salon test. Specifically, the composition of EX. 11 was tested for its ability to cleanse the hair and to impart hair conditioning properties to the shampooed hair. It should be understood that in the subjective salon test, a composition that imparts hair conditioning properties during shampooing equivalent to the conditioning properties imparted by PERT PLUS is considered a premium conditioning because PERT PLUS is recognized in the art as a superior shampoo-conditioner product.

In a standard salon test, the composition of interest is applied to one half of a head of hair, and the composition used for comparison, i.e., PERT PLUS, is applied to the other half of the same head of hair. After shampooing and rinsing, each side of hair is judged for a variety of hair conditioning properties by a trained judge in a subjective ranking of 1 unit (worst) to 5 units (best). Then, the ratings of the judges for each hair conditioning property are averaged, and a difference in rating one half of the hair compared to the other half of the hair of at least 0.3 units is considered a significant difference for that particular hair-conditioning property. The trained judges rate the shampoo and the shampooed hair for such shampooing and hair conditioning properties as ease of application, foam volume, foaming speed, detangling, drying difficulty, fragrance, ease of rinsing, wet feel, wet comb, residue, dry combing, dry feel, coating, flakes/dust, static manageability, condition of ends, sheen/luster, body, effect of hair color, irritation and overall condition.

Accordingly, it was found that, in a salon comparative test between the composition of EX. 11 and PERT PLUS shampoo-conditioner, the composition of EX. 11 performed essentially equally to PERT PLUS in each of the tested properties, including first lather foam volume, second lather foaming speed, ease of application, detangling and ease of drying. For each remaining conditioning property that was rated, the composition of EX. 11 tested only slightly lower in performance than PERT PLUS, i.e. tested within 0.3 units.

Therefore, considering the excellent hair-conditioning properties imparted to hair by PERT PLUS during shampooing, and considering that PERT PLUS includes both an effective cationic conditioning compound and a high performance polydimethylsiloxane conditioning agent, it is both surprising and unexpected for a composition of the present invention (EX. 11), that does not include a high performance polydimethylsiloxane conditioning agent, to impart essentially identical hair conditioning properties to hair during shampooing as the commercial composition. Overall, the comparative salon tests show that a composition of the present invention, including an anionic cleansing surfactant, a polymeric cationic conditioning compound, a cationic conditioning surfactant and a fatty ester, surprisingly and unexpectedly is a metastable hair shampoo-conditioner product that exhibits an extended shelf life, that generates a copious and stable lather, that effectively cleanses the hair, and that imparts excellent hair-conditioning properties to hair during shampooing.

Accordingly, the method and composition of the present invention cleanse the hair and impart a level of physical and cosmetic conditioning properties to hair during shampooing that usually is observed only by treating the hair sequentially, first with a hair shampoo composition, then with a hair conditioning composition. It is both surprising and unexpected for a composition of the present invention, including an anionic cleansing surfactant, a fatty ester and a combination of cationic conditioning compounds to demonstrate such an excellent storage stability, and yet be able to effectively cleanse the hair and to sufficiently deposit the conditioning compounds on the hair to impart such a high degree of conditioning to the shampooed hair.

To even further demonstrate the ability of a composition of the present invention to simultaneously cleanse and condition hair, the hair-shampoo conditioner compositions of EXS. 15-17 were prepared by the above-described method of preparing the compositions of EXS. 11-14. The compositions of EXS. 15-17 then were applied to hair, and the shampooed hair was examined to determine the conditioning properties imparted to the hair by the compositions of the present invention. The weight percentages listed in each of the following examples represent the actual amount of each ingredient present in the hair shampoo-conditioner composition.

| INGREDIENT (by weight) | EX. 15 | EX. 16 | EX. 17 |
|---|---|---|---|
| Quaternized Guar Gum ¹⁾ | 1.50 | 2.00 | 1.50 |
| Ricinoleamidopropyl Trimonium Chloride ²⁾ | 1.65 | 2.42 | 1.65 |
| Linoleamidopropyl PG-Dimonium Chloride Phosphate ³⁾ | 0.60 | 0.60 | 0.60 |
| Cocamide MEA | 2.50 | 1.50 | - |
| Lauramide MEA | - | - | 1.50 |
| Ammonium Lauryl Sulfate | 4.00 | 4.00 | 4.00 |
| Ammonium Lauryl Ether Sulfate (1 mole ethylene oxide) | 4.00 | 4.00 | 4.00 |
| Cocamidopropyl Betaine | 4.00 | 4.00 | 4.00 |
| Cetearyl Octanoate ⁴⁾ | 2.00 | 2.10 | 2.00 |
| Deionized Water | q.s. | q.s. | q.s. |
| Optional Ingredients (dye, fragrance, preservatives, etc.) | q.s. to 100% | q.s. to 100% | q.s. to 100% |

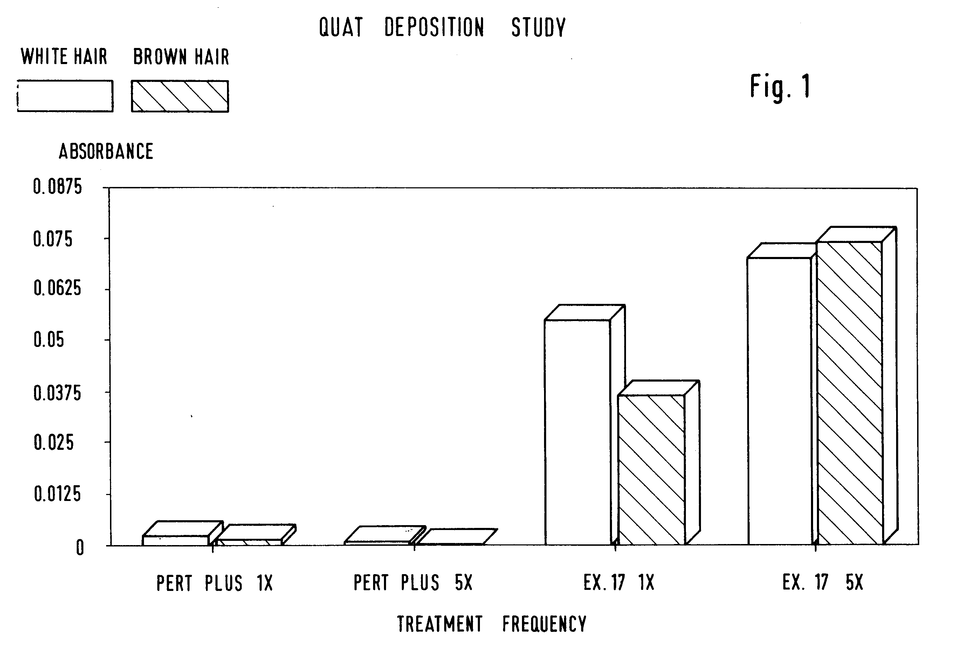

The ability of the compositions of EXS. 15-17 to impart hair conditioning properties to hair during shampooing was investigated by comparing the compositions of EXS. 15-17 to PERT PLUS. The results are illustrated in FIGS. 1 through 8. In general, FIGS. 1 through 8 show that a composition of the present invention imparts hair conditioning properties to hair essentially equal to PERT PLUS, a leading commercial hair shampoo-conditioner recognized in the industry as imparting exceptional hair conditioning properties to hair.

In particular, FIG. 1 illustrates the ability of the composition of EX. 17 to deposit the substantive cationic conditioning compounds on hair. In this test, a hair tress was shampooed either once (1X) or five times (5X) with the hair shampoo-conditioner composition of EX. 17 or with PERT PLUS. The total amount of cationic conditioning compound deposited on the hair was determined by the Fast Pyrazole Red method. The Fast Pyrazole Red method is a standard analytical method, and is well-known to a person of ordinary skill in the art of hair care formulation and testing. In the Fast Pyrazole Red method, the dye binds to the cationic conditioning agent and the dye absorbs incident radiation. Therefore, when testing a treated hair tress, the greater the absorbance value, the greater the amount of cationic conditioning agent that has deposited on the hair.

Accordingly, FIG. 1 shows that a composition of the present invention has a substantially improved ability to deposit the cationic conditioning compounds on both brown hair and white hair, both after one shampooing and after five shampooings. Accordingly, it is expected that hair shampooed with a compound of the present invention would demonstrate better conditioning properties than hair shampooed with PERT PLUS because a composition of the present invention demonstrates an improved ability to deposit the cationic conditioning compounds on the hair.

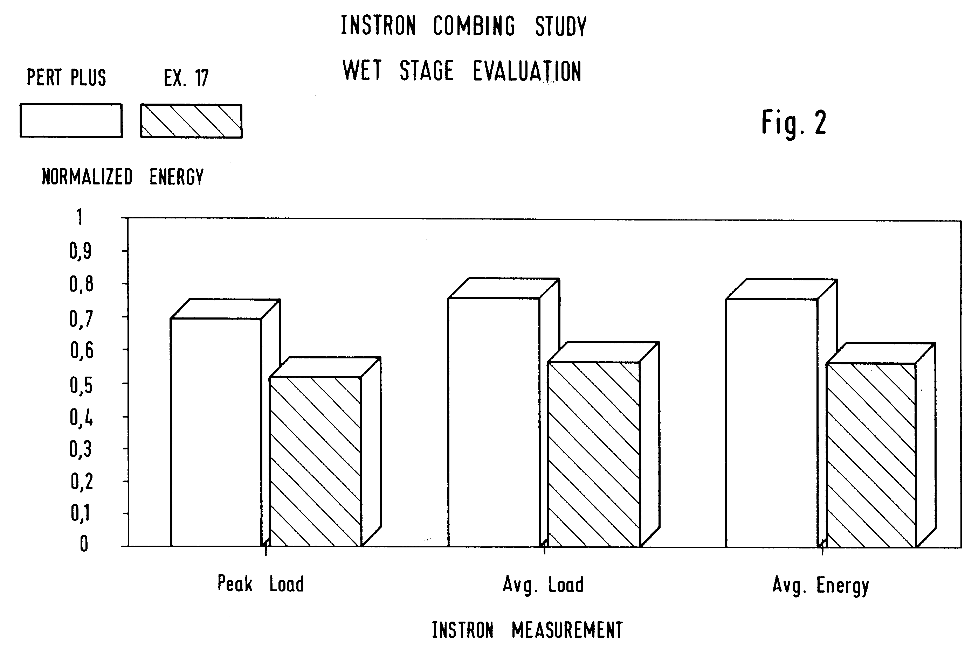

FIG. 2 illustrates that wet hair shampooed with a hair shampoo-conditioner of the present invention is more easily combed than hair shampooed with PERT PLUS. The bar graphs of FIG. 2 show the comparative Instron combing studies between wet hair shampooed with the composition of EX. 17 and wet hair shampooed with PERT PLUS. The Instron combing study measures the force needed to comb through a wet, shampooed hair tress. First, an untreated, wet hair tress is tested and assigned a normalized energy index value of one. Next, a shampooed wet hair tress is tested. If the shampooed hair tress exhibits an index value of less than one, then less energy is required to comb through the shampooed tress than the untreated tress. Therefore, the lower the index value, the more easily the hair is combed, i.e. the hair is more conditioned.

Accordingly, the bar graph in FIG. 2 shows that PERT PLUS, recognized in the industry as a premium hair shampoo-conditioner, demonstrated a wet combing index of about 0.65 to about 0.75. However, the composition of EX. 17 demonstrated a substantially improved wet combing index of from about 0.50 to about 0.55. Therefore, from the bar graphs in FIG. 2, it is observed that wet hair, shampooed with a composition of the present invention, is easier to comb than wet hair shampooed with PERT PLUS because the peak load, average load and average energy are each about 25% less for hair shampooed with a composition of the present invention.

Overall, a composition of the present invention exhibited better wet combing properties than PERT PLUS. Therefore, the cationic conditioning compounds present in the composition of EX. 17 are deposited on the hair shaft more effectively than the conditioning agents present in PERT PLUS, as further demonstrated in the bar graphs of FIG. 1.

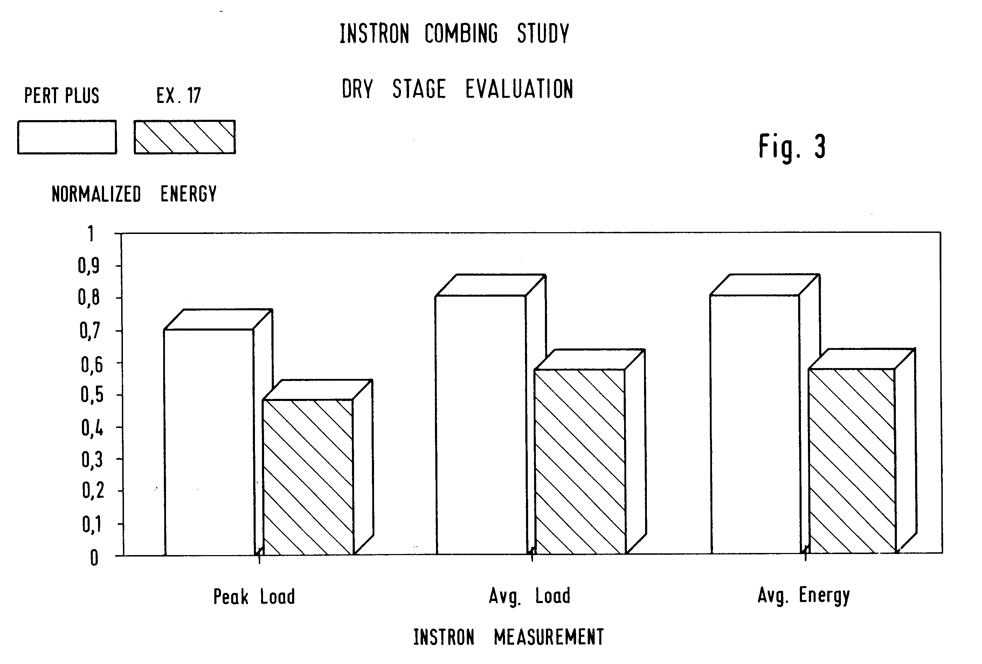

FIG. 3 demonstrates similar comparative results for an Instron combing study on dried shampooed hair. In this test, hair tresses were shampooed either with the composition of EX. 17 or with PERT PLUS. Then the total amount of energy required to comb through the dry, shampooed tress was measured. As in the wet combing test, a lower normalized energy index value signifies easier combing of the dry tress. Similar to the results obtained in the wet tress test, the composition of EX. 17 outperformed PERT PLUS. The hair tress shampooed with the composition of EX. 17 demonstrated normalized energy values of from about 0.48 to about 0.57, whereas hair shampooed with PERT PLUS demonstrated values of from about 0.7 to about 0.8, for an approximately 30% improvement in ease of dry combing.

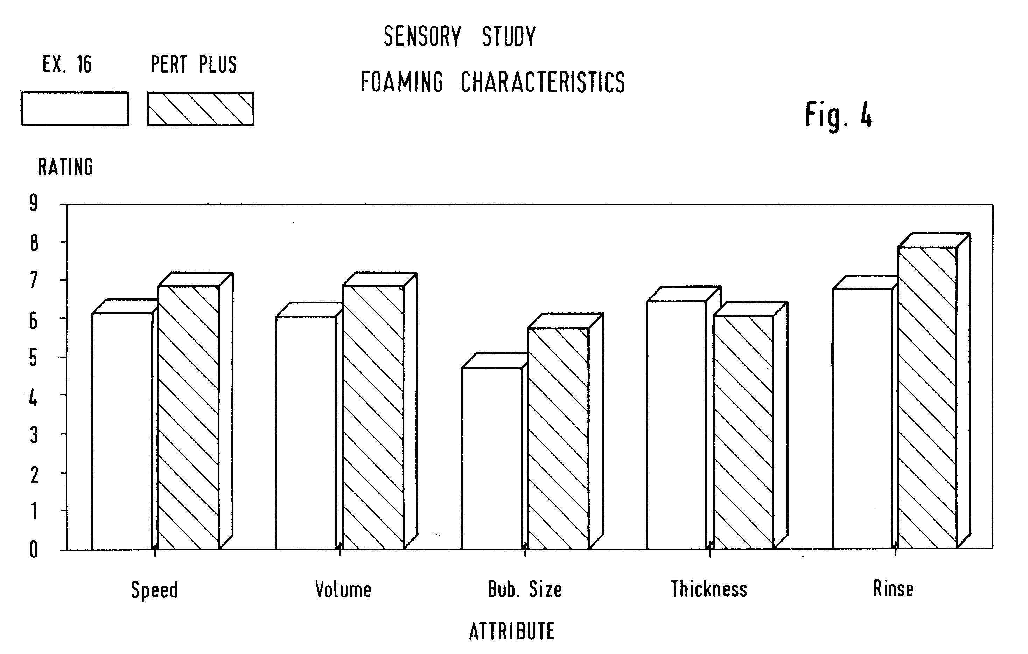

The bar graphs presented in FIG. 4 compare foaming characteristics exhibited by the composition of EX. 16 and exhibited by PERT PLUS. Hair tresses were shampooed either with the composition of EX. 16 or with PERT PLUS. Then, a group of fourteen trained panelists, in a blind test, rated the shampooed tresses for specific foaming properties. The panelists rated hair shampooed with the composition of EX. 16 slightly lower than PERT PLUS for the foaming properties of speed of foaming, foam volume, bubble size and rinsability. The composition of EX. 16 demonstrated a slightly superior foam thickness. Therefore, in general, a composition of the present invention exhibits esthetic properties after application to hair that are essentially similar to the esthetic properties exhibited by PERT PLUS. Such esthetic properties are especially important for consumer acceptance because a consumer equates a copious, stable foam with good hair cleansing efficiency. It should be noted that a composition including greater than 2% of the quaternized guar gum demonstrated a significantly decreased foaming ability; had a slimy feel; and imparted a greasy-feeling buildup on shampooed hair. Therefore, both composition esthetics and composition performance are decreased when the amount of polymeric cationic conditioning compound in the composition exceeds about 2% by weight.

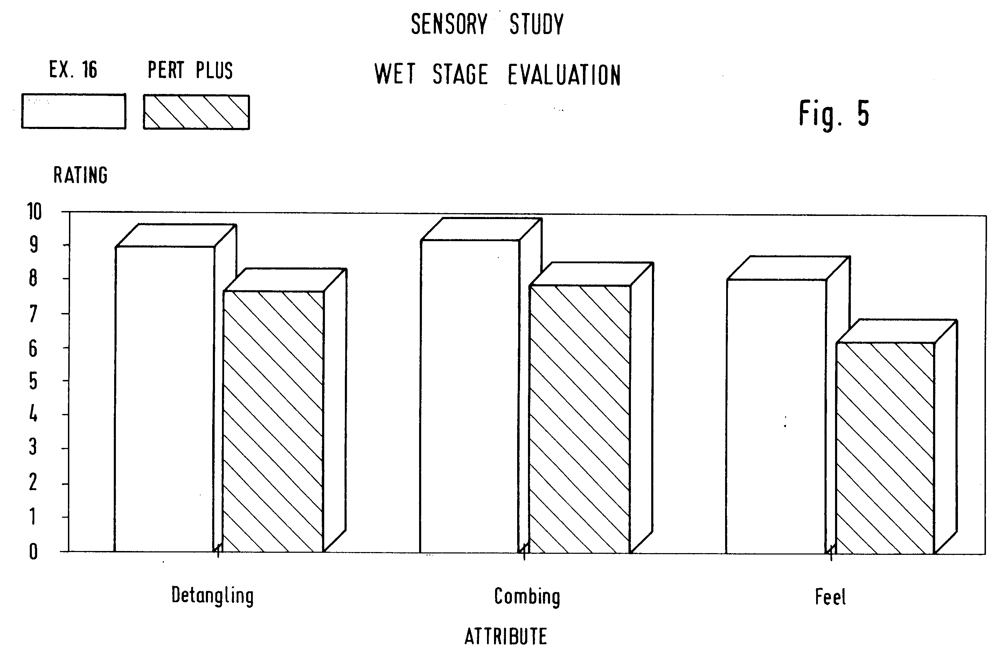

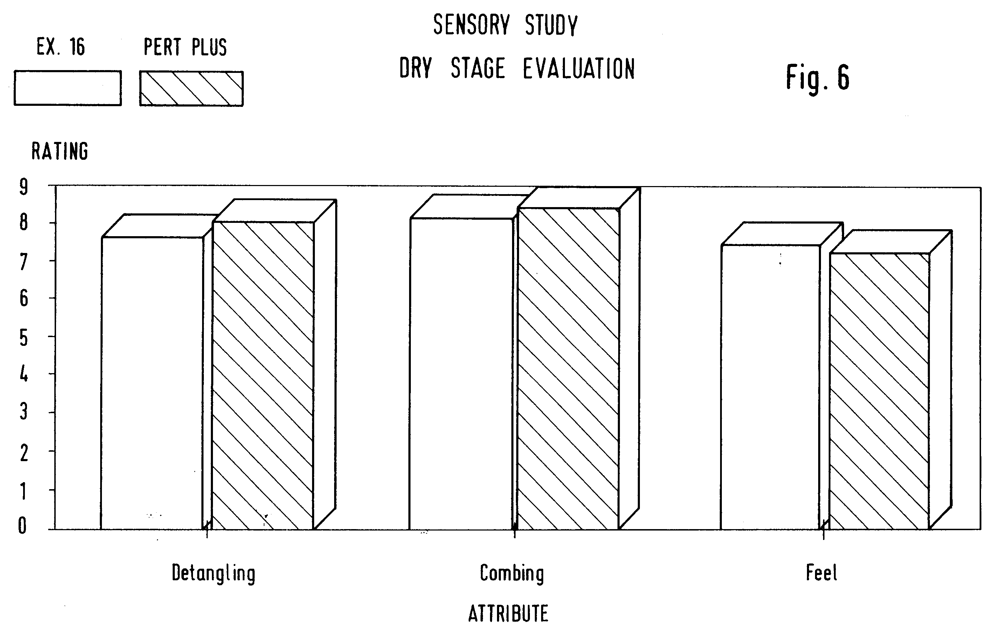

Similarly, the bar graphs in FIGS. 5 and 6 compared the conditioning properties of wet hair (FIG. 5) or dry hair (FIG. 6) shampooed with the composition of EX. 16 to hair shampooed with PERT PLUS. Trained panelists (14) rated the wet hair or the dry hair for the conditioning properties of detangling, combing and feel on a scale of 1 (worst) to 10 (best). In the wet stage, hair shampooed with a composition of the present invention demonstrated improved conditioning properties over hair treated with PERT PLUS. In the dry stage, the composition of EX. 16 and PERT PLUS performed essentially equally. As stated above, PERT PLUS includes both a cationic conditioning compound and a high performance polydimethylsiloxane conditioning agent. Therefore, it is both surprising and unexpected for a composition of the present invention to impart improved wet stage conditioning properties and essentially identical dry stage conditioning properties to shampooed hair as a leading commercial hair shampoo-conditioner composition.

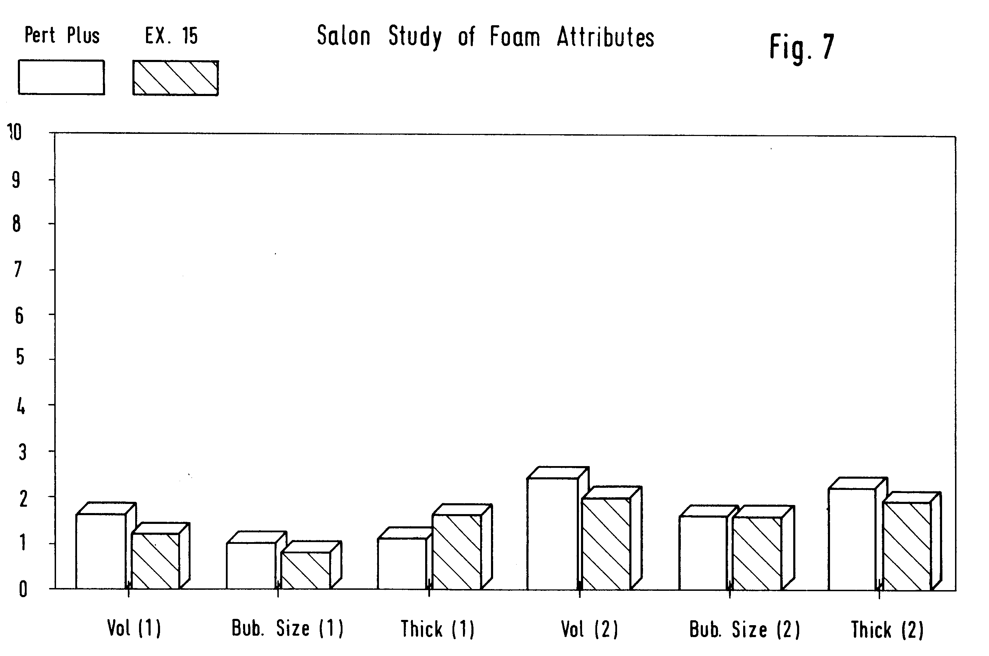

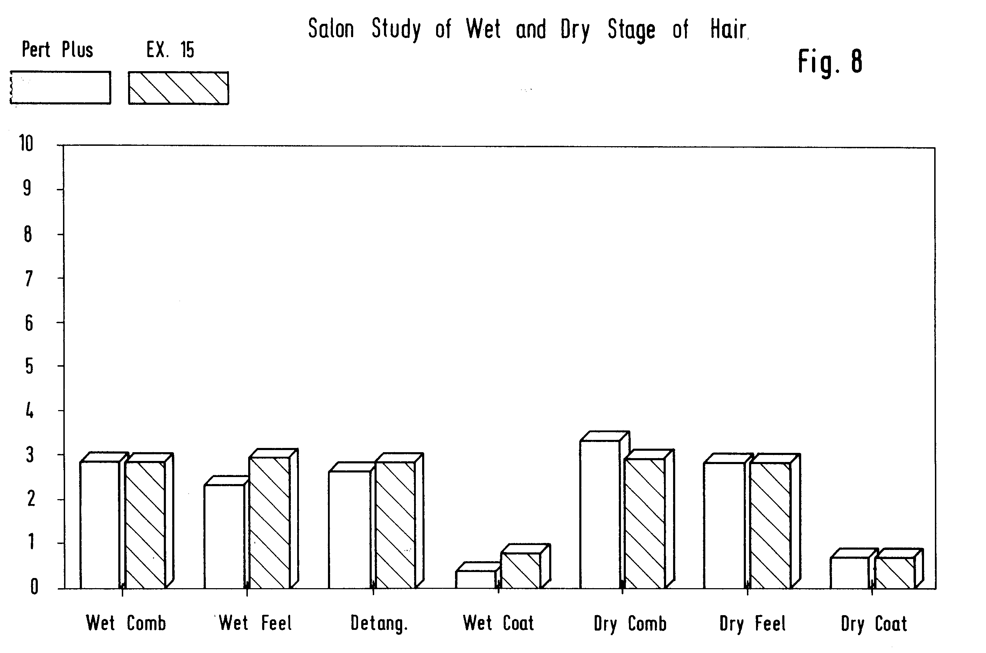

A composition of the present invention (EX. 15) also was compared to PERT PLUS in a salon test for foaming properties. In this comparative test, trained beauticians shampooed one-half of a head with the composition of EX. 15 and the other half with PERT PLUS. The beauticians rated each hair shampoo-conditioner composition for foam volume, bubble size and foam thickness after a first lather, and again after a second lather. The results are illustrated in the bar graphs of FIG. 7, wherein it is demonstrated that the composition of EX. 15 performed essentially equally to PERT PLUS. Similarly, FIG. 8 shows a comparative salon test performed by trained beauticians for various wet and dry conditioning properties imparted to hair shampooed with either the composition of EX. 15 or PERT PLUS. The bar graphs of FIG. 8 illustrate that a composition of the present invention imparts identical or slightly improved wet stage conditioning properties, and, except for dry combing, essentially identical dry stage conditioning properties as PERT PLUS. Therefore, in general, a composition of the present invention imparts both excellent wet combing and excellent dry combing properties to shampooed hair.

It further has been demonstrated that the polymeric cationic conditioning compound, when included in the present hair shampoo-conditioner composition in an amount of greater than about 2% by weight of the composition, imparts an esthetically-unacceptable slimy feel to the composition and a greasy build-up to shampooed hair. Therefore, to demonstrate that a composition of the present invention including a low amount of the polymeric cationic conditioning compound is stable and imparts conditioning properties to treated hair, the compositions of EXS. 18 through 21 were prepared. The compositions of EXS. 18 through 21 each include less than 1% by weight of the polymeric cationic conditioning compound, and each was prepared by the above-described method of preparing the compositions of EXS. 11 through 17. The compositions of EXS. 18 through 21 then were compared to PERT PLUS for an ability to impart improved wet combing and dry combing properties to shampooed hair.

| INGREDIENT (by weight) | EX. 18 | EX. 19 | EX. 20 | EX. 21 |
|---|---|---|---|---|
| Quaternized Guar Gum ¹⁾ | 0.25 | 0.25 | 0.25 | 0.88 |
| Ricinoleamidopropyl Trimonium Chloride ²⁾ | 4.40 | 4.40 | 4.40 | 4.40 |
| Cocamide MEA | 1.00 | 5.00 | 5.00 | 5.00 |
| Linoleamidopropyl PG-Dimonium Chloride Phosphate ³⁾ | 0.60 | 0.60 | 0.60 | 0.60 |
| Ammonium Lauryl Sulfate | 2.68 | 6.43 | 2.68 | 6.43 |
| Ammonium Lauryl Ether Sulfate (1 mole ethylene oxide) | 2.68 | 6.43 | 2.68 | 6.43 |
| Cocamidopropyl Betaine | 3.90 | 4.00 | 1.00 | 2.50 |
| Cetearyl Octanoate ⁴⁾ | 2.00 | 2.00 | 2.00 | 2.00 |
| Optional Ingredients (dye, fragrance, preservatives, etc.) | q.s. | q.s. | q.s. | q.s. |
| Deionized Water | q.s. to 100% | q.s. to 100% | q.s. to 100% | q.s. to 100% |

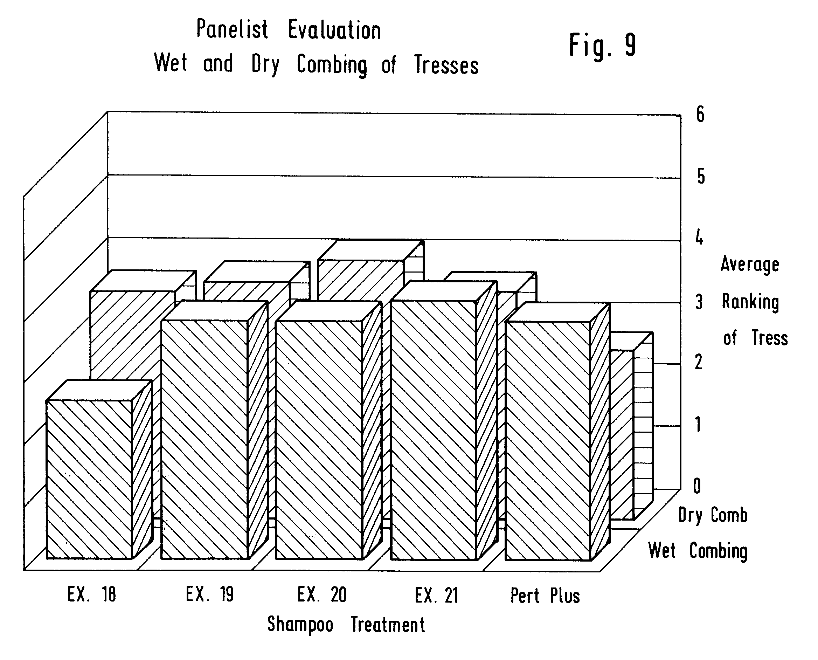

Each composition of EXS. 18 through 21 was compared to PERT PLUS for an ability to impart improved wet combing and dry combing properties to shampooed hair. In general, each of the compositions of EX. 18 through 21 and PERT PLUS was applied to a tress of human hair and the hair was shampooed. After rinsing the shampoo-conditioner composition from the hair, six trained panelists subjectively ranked the shampooed hair tresses on a scale of from 1 (easiest to comb) to 6 (hardest to comb) for both wet combing and dry combing properties. The average ranking for each tress then was calculated. PERT PLUS was included in this comparative test as a control. The results of the comparative test are illustrated in the bar graphs of FIG. 9.

In general, the bar graphs of FIG. 9 show that the compositions of EXS. 18 through 21 compare favorably with the leading competitive hair shampoo-conditioner PERT PLUS for an ability to impart wet combing and dry combing conditioning properties to treated hair. In particular, the composition of EX. 18, including only 0.25% of the quaternized guar gum outperformed PERT PLUS in regards to imparting wet combing properties. Similarly, EXS. 19 through 21 each performed essentially equally to PERT PLUS in regard to imparting wet combing properties. PERT PLUS outperformed the compositions of EXS. 18 through 21 in an ability to impart dry combing properties to treated hair. However, this result is expected due to the presence of a high performance silicone conditioning agent in PERT PLUS. It further should be noted that the compositions of EXS. 18 through 21 nevertheless did impart improved dry combing conditioning properties to hair even at such a reduced amount of quaternized guar gum. Furthermore, the compositions of EXS. 18 through 21 were stable compositions that did not have a slimy feel; that did not impart a greasy feel or build-up to shampooed hair; that generated an acceptable foam level and foam stability, even at the low anionic and nonionic surfactant levels of EX. 18; and that effectively cleansed the hair.

To further demonstrate the ability of a composition of the present invention to impart improved conditioning properties to treated hair, the following compositions of EXS. 22 through 26 were prepared. The compositions of EXS. 22 through 25 each were applied to a human hair tress and were compared to the commercial product PERT PLUS for an ability to impart conditioning properties to the hair. The composition of EX. 26 was prepared to show the stability of a composition including a quaternary ammonium compound of general structural formula (I) as the only cationic conditioning composition. The compositions of EXS. 23 and 24 include only a compound of general structural formula (II) as the cationic conditioning compound. The compositions of EXS. 22 and 25 include a compound of general structural formula (I) and of general structural formula (II) as the cationic conditioning compounds.

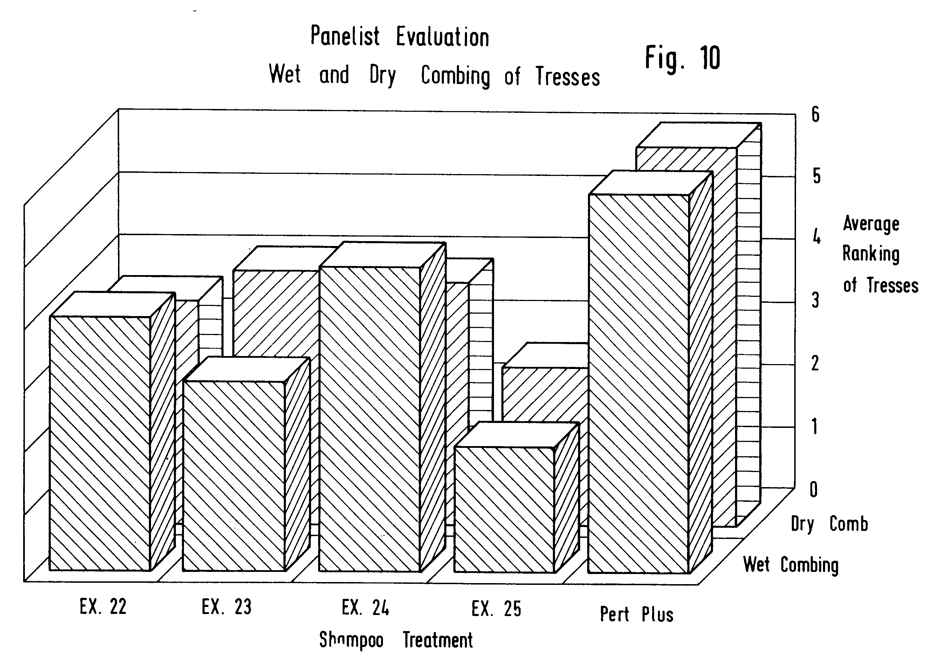

The compositions of EXS. 22 through 26 were prepared by the above-described method of preparing the compositions of EXS. 11 through 21. The results of the comparative tests are illustrated in FIG. 10, wherein, like FIG. 9, six trained panelists ranked the shampooed tresses, with a wet combing or dry combing rank of one being an easy-to-comb tress, whereas a rank of 6 is a hard-to-comb tress. The rankings of the six panelists were averaged and plotted in FIG. 10.

| INGREDIENT (by weight) | EX. 22 | EX. 23 | EX. 24 | EX. 25 | EX. 26 |
|---|---|---|---|---|---|
| Quaternized Guar Gum ¹⁾ | 0.25 | 0.88 | 1.50 | 1.50 | 2.00 |
| Ricinoleamidopropyl Trimonium Chloride ²⁾ | 4.40 | - | - | 4.40 | 2.37 |
| Cocamide MEA | 1.00 | 1.00 | 1.00 | 5.00 | - |
| Linoleamidopropyl PG-Dimonium Chloride Phosphate ³⁾ | 0.60 | 0.60 | 0.60 | 0.60 | - |
| Ammonium Lauryl Sulfate | 4.55 | 4.55 | 6.43 | 2.68 | 11.99 |
| Ammonium Lauryl Ether Sulfate (1 mole ethylene oxide) | 4.55 | 4.55 | 6.43 | 2.68 | - |
| Cocamidopropyl Betaine | 1.00 | 4.00 | 1.00 | 4.00 | - |
| Cetearyl Octanoate ⁴⁾ | 2.00 | 2.00 | 2.00 | 2.00 | 2.00 |
| Optional Ingredients (dye, fragrance, preservatives, etc.) | q.s. | q.s. | q.s. | q.s. | q.s. |
| Deionized Water | q.s. to 100% | q.s. to 100% | q.s. to 100% | q.s. to 100% | q.s. to 100% |

FIG. 10 illustrates that including a variable amount of the quaternized guar gum (0.25% in EX. 22 to 1.50% in EX. 24) provides a hair shampoo-conditioner of the present invention that imparts improved wet combing and improved dry combing properties to shampooed hair. Surprisingly, the combing properties imparted by each of the compositions of EXS. 22 through 25 are improved over the properties imparted by PERT PLUS, a premium hair shampoo-conditioner composition. For example, hair tresses shampooed with PERT PLUS had an average ranking of greater than 5, whereas the average ranking for hair tresses shampooed with a composition of EXS. 22-25 had an average ranking of no greater than less than 4.

Furthermore, FIG. 10 shows that the composition of EX. 22, including a total of 5.00% by weight of a combination of cationic conditioning surfactants, e.g. SURFACTOL Q1 and PHOSPHOLIPID EFA, performed essentially equally to the composition of EX. 23 that includes only 0.60% total weight of a single cationic conditioning surfactant, e.g. PHOSPHOLIPID EFA. Therefore, it has been demonstrated that including either a fatty amidoalkyl substituted quaternary ammonium compound or a quaternized phosphate ester or a combination thereof, in a composition of the present invention, improves the conditioning properties imparted to shampooed hair.

Each composition of EXS. 22 through 25 was a stable emulsion that did not separate after a prolonged storage period. Similarly, the composition of EX. 26 was a stable emulsion. Each composition of EXS. 22 through 25 also effectively cleansed the tress and did not impart a greasy feel or build-up to the shampooed tress. Overall therefore, from EXS. 18-26 and FIGS. 9 and 10, it has been demonstrated that a composition of the present invention, including the essential ingredients within the disclosed ranges, either equalled or outperformed PERT PLUS in regard to imparting wet stage and dry stage conditioning properties to hair.

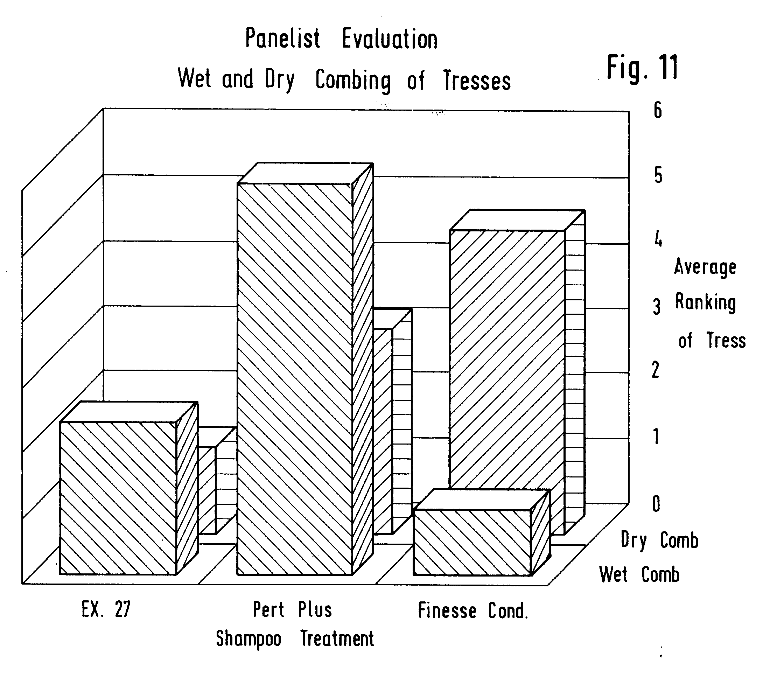

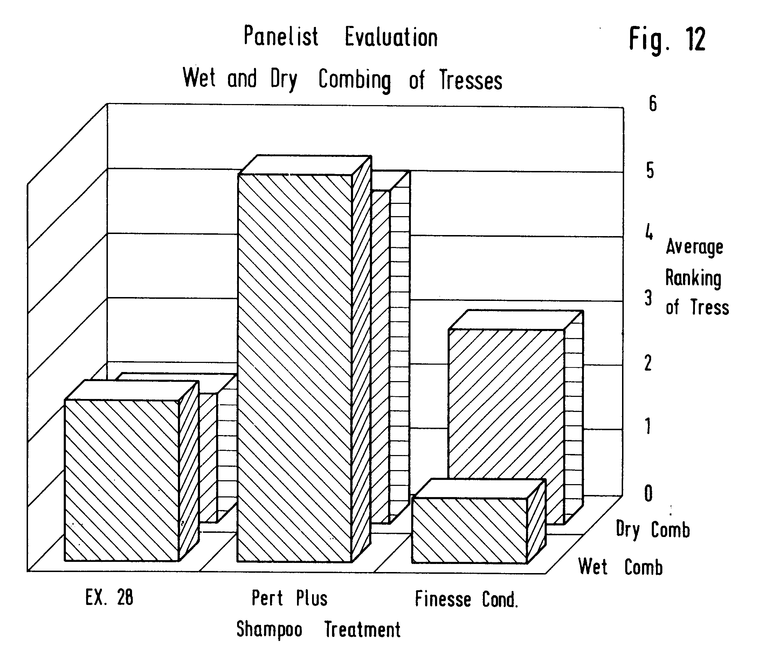

To further demonstrate the ability of a composition of the present invention to impart hair conditioning properties to treated hair, the compositions of EXS. 27 and 28 were prepared, then compared to PERT PLUS shampoo-conditioner and to FINESSE hair conditioner. As previously stated PERT PLUS is recognized in the industry as an effective shampoo having the ability to impart exceptional hair conditioning properties to hair. Similarly, FINESSE, commercially-available from Helene Curtis, Inc., Chicago, IL., is a conditioning compound that is recognized in the industry as imparting superlative hair conditioning properties to treated hair. It is well-known in the art that a hair conditioning composition generally imparts more conditioning properties to treated hair than a hair shampoo-conditioning composition. The results of the comparative tests are illustrated in FIGS. 11 and 12, using the same average ranking scheme described-above in regard to FIGS. 9 and 10.

| INGREDIENT (by weight | EX. 27 | EX. 28 |
|---|---|---|
| Quaternized Guar Gum ¹⁾ | 1.50 | 1.00 |
| Ricinoleamidopropyl Trimonium Chloride ²⁾ | 4.40 | 1.92 |
| Cocamide MEA | 1.00 | 1.00 |
| Linoleamidopropyl PG-Dimonium Chloride Phosphate ³⁾ | 0.60 | 0.60 |
| Ammonium Lauryl Sulfate | 6.43 | 4.50 |
| Ammonium Lauryl Ether Sulfate (1 mole ethylene oxide) | 6.43 | 4.50 |
| Cocamidopropyl Betaine | 4.00 | 3.00 |
| Cetearyl Octanoate ⁴⁾ | 2.00 | 2.00 |
| Optional Ingredients (dye, fragrance, preservatives, etc.) | q.s. | q.s. |
| Deionized Water | q.s. to 100% | q.s. to 100% |

FIG. 11 illustrates the comparative test between the composition of EX. 27, PERT PLUS and FINESSE. From FIG. 11, it is observed that the composition of EX. 27, with an average ranking of less than 2, significantly outperformed the PERT PLUS hair shampoo-conditioner (average ranking greater than 5) and performed almost as well as FINESSE conditioner (average ranking about 1) in wet combing. Surprisingly, the composition of EX. 27 significantly outperformed PERT PLUS and FINESSE in dry combing properties. Such a result is unexpected because the composition of EX. 27 does not include the high performance silicone conditioners that are present in PERT PLUS and FINESSE.

FIG. 12 shows substantially similar results in a comparative test between the composition of EX. 28, PERT PLUS and FINESSE. However, the results illustrated in FIG. 12 are even more surprising because the composition of EX. 28 significantly outperforms PERT PLUS in wet combing and dry combing, and outperforms FINESSE in dry combing, even though the composition of EX. 28 includes only one-half the amount of cationic conditioning compounds as the composition of EX. 27 (i.e. 5.00% in EX. 27 and 2.52% in EX. 28) and only two-thirds of the amount of polymeric cationic conditioning compound (i.e. 1.50% in EX. 27 and 1.00% in EX. 28). The composition of EX. 28 also effectively cleansed the shampooed tress, even though the composition of EX. 28 included 30% less anionic cleansing surfactant than the composition of EX. 27. The compositions of EXS. 27 and 28 were stable emulsions having a viscosity suitable for a hair shampoo-conditioner composition.

In addition to the above features, the method and composition of the present invention provide the further benefits of not leaving the hair tacky or sticky; imparting body and shine to shampooed hair; not leaving the hair with an oily or greasy appearance; not forming a crust and therefore providing combability; and providing manageable and styleable hair having body. Furthermore, after shampooing the hair feels natural and thickened, has body, is soft, shiny, manageable, and combable. The composition of the present invention also conditions the scalp as it conditions the hair.

## Claims

1. A hair shampoo-conditioner composition comprising:
(a) from 1 % to 15 % by weight of an anionic cleansing surfactant;
(b) from 0.1 % to 2 % by weight of a polymeric cationic conditioning compound having an average molecular weight of at least 100,000;
(c) from 0.2 % to 10 % by weight of a cationic conditioning surfactant;
(d) from 0.1 % to 3 % by weight of a nonionic fatty ester; and
(e) a carrier comprising water.

2. The composition of claim 1 wherein the anionic cleansing surfactant has a hydrophobic carbon chain having from eight up to and including 30 carbon atoms, and a hydrophilic moiety selected from the group consisting of sulfate, sulfonate, carbonate, phosphate, carboxylate and mixtures thereof.

3. The composition of claim 2 wherein the hydrophobic carbon chain is etherified with from one mole to four moles of ethylene oxide, propylene oxide or a mixture thereof.

4. The composition of any one of claims 1 to 3 wherein the anionic cleansing surfactant is an alkali metal salt, an ammonium salt, an alkylammonium salt or a hydroxyalkylammonium salt, wherein the alkyl group has from one up to and including three carbon atoms, of an alkyl sulfate, an alkyl ether sulfate, an alkyl ether sulfonate, a sulfate ester of an alkylphenoxy polyoxyethylene ethanol, an alpha-olefin sulfonate, a beta-alkyloxy alkane sulfonate, an alkyl arylsulfonate, an alkyl carbonate, an alkyl ether carboxylate, a fatty acid, a sulfosuccinate, a sarcosinate, an octoxynol phosphate, a nonoxynol phosphate, a taurate, a fatty tauride, a sulfated monoglyceride, a fatty acid amido polyoxyethylene sulfate, an isothienate or mixtures thereof, wherein the fatty moiety has from twelve up to and including eighteen carbon atoms and the alkyl moiety has from twelve up to and including eighteen carbon atoms, preferably of lauryl sulfate, dodecylbenzenesulfonate, lauryl sulfosuccinate, a lauryl ether sulfate having from one up to and including 4 moles of ethylene oxide, a lauryl ether carboxylate having from one up to and including 4 moles to ethylene oxide, lauryl sarcosinate, cocomethyl tauride, a sulfosuccinate half ester amide and mixtures thereof.

5. The composition of any one of claims 1 - 4 wherein the anionic cleansing surfactant is present in an amount ranging from 3 % to 12 %, preferably from 9 % to 12 % by weight of the composition.

6. The composition of any one of claims 1 - 5 wherein the polymeric cationic conditioning compound has an average molecular weight in the range of from 200,000 to 1,000,000, preferably in the range of from 250,000 to 750,000.

7. The composition of anyone of claims 1 - 6 wherein the polymeric cationic conditioning compound comprises a synthetic quaternized polymer which is preferably selected from the group consisting of polyquaternium-1, polyquaternium-2, polyquaternium-5, polyquaternium-6, polyquaternium-7, polyquaternium-8, polyquaternium-9, polyquaternium-11, polyquaternium-12, polyquaternium-13, polyquaternium-14 polyquaternium-15 and mixtures thereof.

8. The composition of any one of claims 1 - 6 wherein the polymeric cationizing compound comprises a naturally-derived quaternized polymer which is preferably selected from the group consisting of polyquaternium-4, polyquaternium-10, guar hydroxypropyltrimonium chloride and mixtures thereof.

9. The composition of any one of claims 1 - 8 wherein the polymeric cationic conditioning compound is present in the range of from 0.2 % to 1 % by weight of the composition.

10. The composition of claim 1 wherein the cationic conditioning surfactant having a phosphate ester moiety has the structural formula: 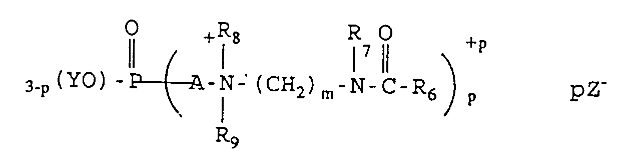 wherein R₆ is an aryl group, an alkaryl group, a saturated alkyl group, an unsaturated alkyl group, a saturated hydroxyalkyl group or an unsaturated hydroxyalkyl group, wherein the alkyl or hydroxyalkyl group has from seven up to and including 21 carbon atoms; R₇ is hydrogen, an alkyl group or a hydroxyalkyl group having from one up to and including six carbon atoms; R₈ and R₉, independently, are an alkyl group or a hydroxyalkyl group having from one up to and including six carbon atoms; A is a residue of a glycol or a triol having from two up to and including four carbon atoms; Z is an anion selected from the group consisting of chloride, bromide, methosulfate, ethosulfate, and mixtures thereof; m is a numeral from one to 10; Y is selected from the group consisting of hydrogen, an alkyl group, a hydroxyalkyl group and an aryl group, wherein the alkyl or the hydroxyalkyl group has from one up to and including 22 carbon atoms; and p is a number from 1 to 3, wherein R₆ preferably is the alkyl moiety of an essential fatty acid, in particular of linoleic acid, arachidonic acid, ricinoleic acid and mixtures thereof.

11. The composition of claim 10 wherein p is 3.

12. The composition of claims 10 or 11 wherein the cationic surfactant is a tri(C₈-C₂₂ alkamidopropyl PG-dimonium chloride), preferably linoleamidopropyl PG-dimonium chloride phosphate.

13. The composition of any one of claims 1 - 11 wherein the cationic conditioning surfactant is present in an amount ranging from 0.5 % to 5 % by weight of the composition.

14. The composition of any one of claims 1 - 13 wherein the fatty ester is present in an amount ranging from 0.5 % to 3 % by weight of the composition.

15. The composition of any one of claims 1 - 14 wherein the fatty ester comprises a carboxylic acid having from six up to and including 12 carbon atoms esterified with a fatty alcohol having from 12 up to and including 22 carbon atoms, which is preferably selected from the group consisting of lauryl alcohol, myristyl alcohol, cetyl alcohol, cetearyl alcohol, stearyl alcohol, isostearyl alcohol, oleyl alcohol, tallow alcohol, behenyl alcohol and mixtures thereof.

16. The composition of claim 15 wherein the fatty ester is selected from the group consisting of cetyl octanoate, cetearyl octanoate, stearyl heptanoate, stearyl caprylate, stearyl octanoate, lauryl octanoate, myristyl heptanoate, oleyl octanoate and mixtures thereof.

17. The composition of any one of claims 1 - 14 wherein the fatty ester comprises a carboxylic acid having from 8 up to and including 22 carbon atoms esterified with an alcohol having from 1 up to and including 6 carbon atoms, the fatty ester being preferably selected from the group consisting of isopropyl myristate, isopropyl palmitate, isopropyl laurate, isopropyl linoleate, isopropyl isostearate, isopropyl oleate, isopropyl stearate, isopropyl tallowate, isopropyl ricinoleate, methyl laurate, methyl linoleate, methyl myristate, methyl stearate, methyl ricinoleate, methyl caprylate, methyl oleate, methyl palmitate, methyl stearate, methyl behenate, methyl soyate, methyl tallowate, isopropyl behenate, isopropyl soyate, propyl oleate, butyl oleate, butyl stearate, methyl coconate, methyl lardate, isobutyl palmitate, butyl myristate, ethyl palmitate, ethyl myristate, ethyl oleate, ethyl stearate, isobutyl stearate, isobutyl myristate and mixtures thereof.

18. The composition of any one of claims 1 - 14 wherein the fatty ester comprises an ester of benzoic acid wherein the benzoic acid is esterified with an esterifying alcohol having from eight up to and including 22 carbon atoms, the ester of benzoic acid being preferably selected from the group consisting of benzoic acid esterified with a fatty alcohol including from 12 to 15 carbon atoms, isostearyl benzoate, PPG-15 stearyl ether benzoate and mixtures thereof.

19. The composition of any one of claims 1 - 18 further comprising from 0 % to 5 % by weight of an amphoteric surfactant, from 0 % to 5 % by weight of a nonionic surfactant or a mixture thereof.

20. The composition of claim 19 wherein the amphoteric surfactant is selected from the group consisting of a betaine, a hydroxypropylsultaine, an amine oxide and mixtures thereof, preferably from the group consisting of cocamidopropyl betaine, lauramidopropyl betaine, coco/oleamidopropyl betaine, coco betaine, oleyl betaine, cocamidopropyl hydroxysultaine, tallowamidopropyl hydroxysultaine and dihydroxyehtyl tallow glycinate, or mixtures thereof.

21. The composition of claim 19 wherein the nonionic surfactant is selected from the group consisting of an ester of a polyol, an ester of a sugar, a fatty acid alkanolamide, a polyethylene glycol, an ethoxylated or a propoxylated fatty alcohol, a condensation product of ethylene oxide with a long chain amide and mixtures thereof.

22. The composition of claim 19 wherein the nonionic surfactant is a fatty alkanolamide selected from the group consisting of cocamide MEA, cocamide DEA, soyamide DEA, lauramide DEA, oleamide MIPA, stearamide MEA, myristamide DEA, stearamide DEA, oleylamide DEA, tallowamide DEA, lauramide MIPA, tallowamide MEA, isostearamide DEA, isostearamide MEA and mixtures thereof.

23. The composition of claim 1 wherein the anionic cleansing surfactant is an alkali metal salt, an ammonium salt, an alkylammonium salt or a hydroxyalkylammonium salt, wherein the alkyl group has from one up to and including three carbon atoms, of an alkyl sulfate, an alkyl ether sulfate or a mixture thereof; the polymeric cationic conditioning compound is guar hydroxyproplytrimonium chloride; the cationic conditioning surfactant is ricinoleamidopropyl trimonium chloride, linoleamidopropyl PG-dimonium chloride phosphate or a mixture thereof; and the fatty ester is cetearyl octanoate, which composition preferably further comprises from 0 % to 5 % by weight of cocamidopropyl betaine; from 0 % to 5 % by weight of a fatty alkanolamide selected from the group consisting of cocamide MEA, linoleamide DEA and lauramide DEA; or a mixture thereof.

24. A hair shampoo-conditioner composition comprising:
(a) from 3 % to 15 % by weight of an anionic cleansing surfactant selected from the group consisting of ammonium lauryl sulfate, sodium lauryl sulfate, ammonium lauryl ether sulfate having 1 mole of ethylene oxide, sodium lauryl ether sulfate having 1 mole of ethylene oxide and mixtures thereof;
(b) from 0.1 % to 1 % by weight of a guar hydroxypropyltrimonium chloride;
(c) from 0.2 % to 10 % by weight of a cationic conditioning compound selected from the group consisting of
i) a quaternary ammonium compound having the structural formula: 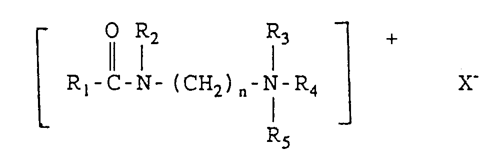 wherein R₁ is a substituted or unsubstituted, saturated or unsaturated, alkyl group having from 5 up to and including 21 carbon atoms; R₂ is hydrogen or methyl; R₃, R₄ and R₅, independently, are methyl, ethyl, hydroxyethyl or benzyl; n is a numeral from one to 10; and X is an anion selected from the group consisting of chloride, bromide, ethosulfate, methosulfate, acetate, nitrate, tosylate, phosphate and mixtures thereof;
ii) a quaternized phosphate ester having the structural formula: 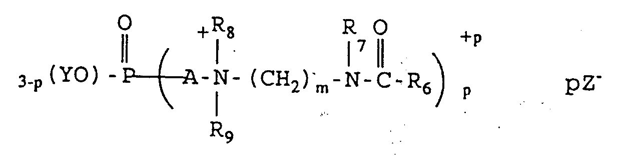 wherein R₆ is an aryl group, an alkaryl group, a saturated or unsaturated alkyl group, or a saturated or unsaturated hydroxyalkyl group wherein the alkyl or hydroxyalkyl group has from about seven up to and including 21 carbon atoms; R₇ is hydrogen, or an alkyl or a hydroxyalkyl group having from one up to and including six carbon atoms; R₈ and R₉, independently, are an alkyl or a hydroxyalkyl group having from one up to and including six carbon atoms; A is a residue of a glycol or a triol having from two up to and including four carbon atoms; Z is an anion selected from the group consisting of chloride, bromide, methosulfate, ethosulfate and mixtures thereof; m is a numeral from one to 10; Y is selected from the group consisting of hydrogen, an alkyl group, a hydroxyalkyl group and an aryl group, either substituted or unsubstituted, and wherein the alkyl or the hydroxyalkyl group has from one up to and including 22 carbon atoms; and p is a number from 1 to 3; and
iii) a mixture thereof;
(d) from 0.1 % to 3 % by weight of a fatty ester selected from the group consisting of cetearyl octanoate, isostearyl benzoate, a fatty (C₁₂-C₁₅) alcohol benzoate and mixtures thereof;
(e) from 0 % to 5 % by weight of an amphoteric surfactant selected from the group consisting of cocamidopropyl betaine, lauramidopropyl betaine, coco/oleamidopropyl betaine, coco betaine, oleyl betaine, cocamidopropyl hydroxysultaine, tallowamidopropyl hydroxysultaine, dihydroxyethyl tallow glycinate and mixtures thereof;
(f) from 0 % to 5 % by weight of a nonionic alkanolamide selected from the group consisting of cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA and mixtures thereof; and
(g) a carrier comprising water.

25. The composition of claim 24 wherein the cationic conditioning compound is selected from the group consisting of ricinoleamidopropyl trimonium chloride, ricinoleamido trimonium ethyl sulfate, hydroxy stearamidopropyl trimonium methyl sulfate and hydroxy stearamidpropyl trimonium chloride, ricinoleamidopropyl ethyldimonium ethosulfate, isostearamidopropyl ethyldimonium ethosulfate, Quaternium-22, Quaternium-26, linoleamidopropyl PG-dimonium chloride phosphate and mixtures thereof.

26. A method of treating hair to simultaneously cleanse the hair and impart conditioning properties to the hair comprising contacting the hair with a composition of any one of claims 1 - 25; and thereafter rinsing the hair.

## Patentansprüche

1. Haarshampoo-Konditionier-Zusammensetzung, enthaltend:
(a) 1 bis 15 Gew.-% eines anionischen Reinigungs-Tensids;
(b) 1 bis 2 Gew.-% einer polymeren kationischen Konditionier-Verbindung mit einem durchschnittlichen Molekulargewicht von mindestens 100.000;
(c) 2 bis 10 Gew.-% eines kationischen Konditionierungs-Tensids;
(d) 0,1 bis 3 Gew.-% eines nichtionischen Fettsäureesters und
(e) einen Träger, enthaltend Wasser.

2. Zusammensetzung nach Anspruch 1, worin das anionische Reinigungs-Tensid eine hydrophobe Kohlenstoffkette mit 8 bis einschließlich 30 Kohlenstoffatomen und eine hydrophile Gruppierung, ausgewählt aus der Gruppe, bestehend aus Sulfat, Sulfonat, Carbonat, Phosphat, Carboxylat und deren Gemischen, enthält.

3. Zusammensetzung nach Anspruch 2, worin die hydrophobe Kohlenstoffkette mit 1 bis 4 Mol Ethylenoxid, Propylenoxid oder deren Gemischen verethert ist.

4. Zusammensetzung nach einem der Ansprüche 1 bis 3, worin das anionische Reinigungs-Tensid ein Alkalisalz, ein Ammoniumsalz, ein Alkylammoniumsalz oder ein Hydroxyalkylammoniumsalz (worin die Alkylgruppe 1 bis einschließlich 3 Kohlenstoffatome enthält) eines Alkylsulfats, eines Alkylethersulfats, eines Alkylethersulfonats, eines Sulfatesters eines Alkylphenoxy-Polyoxyethylen-Ethanols, eines α-Olefinsulfonats, eines β-Alkoxyalkan-Sulfonats, eines Alkyl-Arylsulfonats, eines Alkylcarbonats, eines Alkyl-Ethercarboxylats, einer Fettsäure, eines Sulfosuccinats, eines Sarcosinats, eines Octoxynolphosphats, eines Monoxynolphosphats, eines Taurats, eines Fettsäuretaurids, eines sulfatierten Monoglycerids, eines Fettsäure-Amido-Polyethylen-Sulfats, eines Isothienats oder deren Gemische ist, worin die Fettsäure-Gruppierung 12 bis einschließlich 18 Kohlenstoffatome und die Alkylgruppierung 12 bis einschließlich 18 Kohlenstoffatome enthält, vorzugsweise des Laurylsulfats, Dodecyl-Benzolsulfonats, Lauryl-Sulfosuccinats, eines Laurylethersulfats mit 1 bis einschließlich 4 Mol Ethylenoxid, eines Laurylethercarboxylats mit 1 bis einschließlich 4 Mol Ethylenoxid, eines Laurylsarcosinats, Cocomethyltaurids, eines Sulfosuccinats-Halbesteramids und deren Gemischen.

5. Zusammensetzung nach einem der Ansprüche 1 bis 4, worin das anionische Reinigungstensid in einer Menge von 3 bis 12%, vorzugsweise von 9 bis 12 Gew.%, der Zusammensetzung vorhanden ist.

6. Zusammensetzung nach einem der Ansprüche 1 bis 5, worin die polymere kationische Konditionierverbindung ein durchschnittliches Molekulargewicht im Bereich von 200.000 bis 1.000.000, vorzugsweise im Bereich von 250.000 bis 750.000 hat.

7. Zusammensetzung nach einem der Ansprüche 1 bis 6, worin die polymere kationische Konditionierverbindung ein synthetisches quaternisiertes Polymer darstellt, das vorzugsweise aus der Gruppe, bestehend aus Polyquaternium-1, Polyquaternium-2, Polyquaternium-5, Polyquaternium-6, Polyquaternium-7, Polyquaternium-8, Polyquaternium-9, Polyquaternium-11, Polyquaternium-12, Polyquaternium-13, Polyquaternium-14, Polyquaternium-15 und deren Gemischen ausgewählt ist.

8. Zusammensetzung nach einem der Ansprüche 1 bis 6, worin die polymere kationisierende Verbindung- ein von einem Naturprodukt abgeleitetes quaternisiertes Polymer darstellt, das vorzugsweise aus der Gruppe, bestehend aus Polyquaternium-4, Polyquaternium-8, Guar-Hydroxypropyltrimoniumchlorid und deren Gemischen ausgewählt ist.

9. Zusammensetzung nach einem der Ansprüche 1 bis 8, worin die polymere kationische Konditionierverbindung im Bereich von 0,2 bis 1 Gew.-% der Zusammensetzung vorhanden ist.

10. Zusammensetzung nach Anspruch 1, worin das kationische Konditionier-Tensid mit einer Phosphat-Gruppierung die Strukturformel 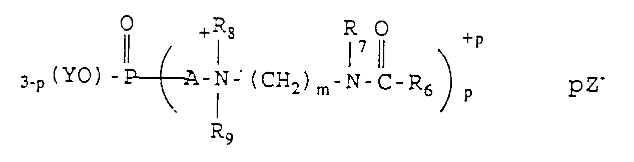 hat, worin R₆ eine Arylgruppe, eine Alkarylgruppe, eine gesättigte Alkylgruppe, eine ungesättigte Alkylgruppe, eine gesättigte Hydroxalkylgruppe oder eine ungesättigte Hydroxyalkylgruppe darstellt, worin die Alkyl- oder Hydroxyalkylgruppe 7 bis einschließlich 21 Kohlenstoffatome enthält; R₇ Wasserstoff, eine Alkylgruppe oder eine Hydroxyalkylgruppe mit 1 bis einschließlich 6 Kohlenstoffatomen darstellen; R₈ und R₉ unabhängig voneinander eine Alkylgruppe oder eine Hydroxyalkylgruppe mit 1 bis einschließlich 6 Kohlenstoffatomen darstellen; A einen Rest eines Glykols oder eines Triols mit 2 bis einschließlich 4 Kohlenstoffatomen darstellt; Z ein Anion aus der Gruppe, bestehend aus Chlorid, Bromid, Methosulfat, Ethosulfat und deren Gemischen, ausgewählt ist; m eine Zahl von 1 bis 10 bedeutet; Y aus der Gruppe, bestehend aus Wasserstoff, einer Alkylgruppe, einer Hydroxyalkylgruppe und einer Arylgruppe ausgewählt ist, worin die Alkyl- oder Hydroxyalkylgruppe 1 bis einschließlich 22 Kohlenstoffatome enthält; und p eine Zahl von 1 bis 3 bedeutet, wobei R₆ vorzugsweise die Alkylgruppe einer essentiellen Fettsäure, insbesondere von Linolsäure, Arachidonsäure, Ricinolsäure und deren Gemischen darstellt.

11. Zusammensetzung nach Anspruch 10, worin p = 3.

12. Zusammensetzung nach Anspruch 10 oder 11, worin das kationische Tensid ein Tri(C₈-C₂₂ Alkamidopropyl-PG-Dimoniumchlorid), vorzugsweise Linoleamidopropyl-PG-Dimonium-Chlorid-Phosphat darstellt.

13. Zusammensetzung nach einem der Ansprüche 1 bis 11, worin das kationische Konditionier-Tensid in einer Menge von 0,5 bis 5 Gew.-% der Zusammensetzung vorhanden ist.

14. Zusammensetzung nach einem der Ansprüche 1 bis 13, worin der Fettsäureester in einer Menge von 0,5 bis 3 Gew.-% der Zusammensetzung vorhanden ist.

15. Zusammensetzung nach einem der Ansprüche 1 bis 14, worin der Fettsäureester eine Carbonsäure mit 6 bis einschließlich 12 Kohlenstoffatomen enthält, die mit einem Fettalkohol mit 12 bis einschließlich 22 Kohlenstoffatomen verestert ist, der vorzugsweise aus der Gruppe, bestehend aus Laurylalkohol, Myristylalkohol, Cetylalkohol, Cetearylalkohol, Stearylalkohol, Isostearylalkohol, Oleylalkohol, Talkalkohol, Behenylalkohol und deren Gemischen ausgewählt ist.

16. Zusammensetzung nach Anspruch 15, worin der Fettsäurester aus der Gruppe, bestehend aus Cetyloctanoat, Cetearyloctanoat, Stearylheptanoat, Stearylcaprylat, Stearyloctanoat, Lauryloctanoat, Myristylheptanoat, Oleyloctanoat und deren Gemischen ausgewählt ist.

17. Zusammensetzung nach einem der Ansprüche 1 bis 14, worin der Fettsäureester eine Carbonsäure mit 8 bis einschließlich 22 Kohlenstoffatomen enthält, die mit einem Alkohol mit 1 bis einschließlich 6 Kohlenstoffatomen verestert ist, wobei der Fettsäureester vorzugsweise aus der Gruppe, bestehend aus Isopropylmiristat, Isopropylpalmitat, Isopropyllaurat, Isopropyllinoleat, Isopropylisostearat, Isopropyloleat, Isopropyltallowat, Isopropylricinoleat, Methyllaurat, Methyllinoleat, Methylmyristat, Methylstearat, Methylricinoleat, Methylcaprylat, Methyloleat, Methylpalmitat, Methylstearat, Methylbehenat, Methylsoyat, Methyltallowat, Isopropylbehenat, Isopropylsoyat, Propyloleat, Butyloleat, Butylstearat, Methylcoconat, Methyllardat, Isobutylpalmitat, Butylmyristat, Ethylpalmitat, Ethylmyristat, Ethyloleat, Ethylstearat, Isobutylstearat, Isobutylmyristat und deren Gemischen ausgewählt ist.

18. Zusammensetzung nach einem der Ansprüche 1 bis 14, worin der Fettsäureester einen Ester der Benzoesäure darstellt, worin die Benzoesäure mit einem veresternden Alkohol mit 8 bis einschließlich 22 Kohlenstoffatomen verestert ist, wobei der Ester der Benzoesäure vorzugsweise aus der Gruppe, bestehend aus Benzoesäure, verestert mit einem Fettalkohol mit 12 bis 15 Kohlenstoffatomen, Isostearylbenzoat, PPG-15 Stearyletherbenzoat und deren Gemischen verestert ist.

19. Zusammensetzung nach einem der Ansprüche 1 bis 18, weiterhin enthaltend 0 bis 5 Gew.-% eines amphoteren Tensids, 0,5 Gew.-% eines nichtionischen Tensids oder eines Gemisches hiervon.

20. Zusammensetzung nach Anspruch 19, worin das amphotere Tensid aus der Gruppe, bestehend aus einem Betain, einem Hydroxypropylsultain, einem Aminoxid und deren Gemischen, vorzugsweise aus der Gruppe bestehend aus Cocoamidopropylbetain, Lauramidopropylbetain, Coco/Oleamidopropylbetain, Cocobetain, Oleylbetain, Cocoamidopropylhydroxysultain, Talg-amidopropyl-Hydroxysultain und Dihydroxyethyltalg-Glycinat oder deren Gemischen, ausgewählt ist.

21. Zusammensetzung nach Anspruch 19, worin das nichtionische Tensid aus der Gruppe, bestehend aus einem Ester eines Polyols, einem Ester eines Zuckers, einem Fettsäurealkanolamid, einem Polyethylenglycol, einem ethoxylierten oder propoxylierten Fettalkohol, einem Kondensationsprodukt von Ethylenoxid mit einem langkettigen Amid oder deren Gemischen ausgewählt ist.

22. Zusammensetzung nach Anspruch 19, worin das nichtionische Tensid ein Fettalkanolamid aus der Gruppe, bestehend aus Cocamid-MEA, Cocamid-DEA, Soyamid-DEA, Lauramid-DEA, Oleamid-MIPA, Stearamid-MEA, Myristamid-DEA, Stearamid-DEA, Oleylamid-DEA, Talgamid-DEA, Lauramid-MIPA, Talgamid-MEA, Isostearamid-DEA, Isostearamid-MEA und deren Gemischen ausgewählt ist.

23. Zusammensetzung nach Anspruch 1, worin das anionische Reinigungs-Tensid ein Alkalisalz, ein Ammoniumsalz, ein Alkylammoniumsalz oder ein Hydroxyalkylammoniumsalz (worin die Alkylgruppe 1 bis einschließlich 3 Kohlenstoffatome enthält) eines Alkylsulfats, eines Alkylethersulfats oder eines Gemisches hiervon darstellt, worin die polymere kationische Konditionierverbindung Guar-Hydroxypropyltrimoniumchlorid darstellt; das kationische Konditionier-Tensid Ricinolamidopropyl-Trimoniumchlorid, Linoleamidopropyl-PG-Dimonium-Chlorid-Phosphat oder ein Gemisch davon darstellt; und der Fettsäureester Cetearyloctanoat darstellt; wobei die Zusammensetzung vorzugsweise zusätzlich 0 bis 5 Gew.-% Cocoamidopropylbetain; 0 bis 5 Gew.-% eines Fettalkanolamids, ausgewählt aus der Gruppe bestehend aus Cocamid-MEA, Linoleamid-DEA und Lauramid-DEA; oder deren Gemisch enthält.

24. Haarshampoo-Konditionier-Zusammensetzung, enthaltend:
(a) 3 bis 15 Gew.-% eines anionischen Reinigungs-Tensids, ausgewählt aus der Gruppe, bestehend aus Ammoniumlaurylsulfat, Natriumlaurylsulfat, Ammoniumlauryl-Ethersulfat mit 1 Mol Ethylenoxid, Natriumlauryl-Ethersulfat mit 1 Mol Ethylenoxid und deren Gemische;
(b) 0,1 bis 1 Gew.-% eines Guar-Hydroxypropyl-Trimoniumchlorids;
(c) 0,2 bis 10 Gew.-% einer kationischen Konditionierverbindung, ausgewählt aus der Gruppe, bestehend aus
(i) einer quaternären Ammmoniumverbindung mit der Strukturformel 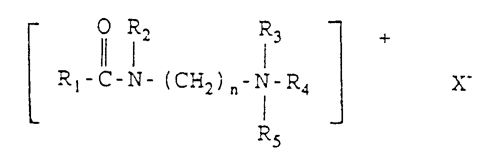 worin R₁ eine substituierte oder unsubstituierte, gesättigte oder ungesättigte Alkylgruppe mit 5 bis einschließlich 21 Kohlenstoffatomen; R₂ Wasserstoff oder Methyl;
R₃, R₄ und R₅ unabhängig voneinander Methyl, Ethyl, Hydroxyethyl oder Benzyl; n eine Zahl von 1 bis 10; und
X ein Anion, ausgewählt aus der Gruppe, bestehend aus Chlorid, Bromid, Ethosulfat, Methosulfat, Acetat, Nitrat, Tosylat, Phosphat und deren Gemischen, darstellen;
ii) einem quaternisierten Phosphatester mit der Strukturformel 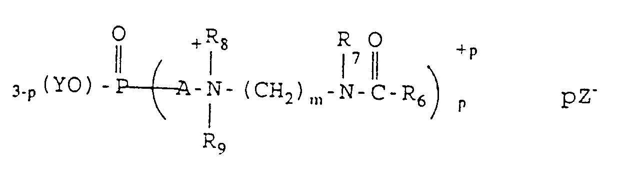 worin R₆ eine Arylgruppe, eine Alkarylgruppe, eine gesättigte oder ungesättigte Alkylgruppe oder eine gesättige Hydroxyalkylgruppe, worin die Alkyl- oder Hydroxyalkylgruppe etwa 7 bis einschließlich 21 Kohlenstoffatome enthält; R₇ Wasserstoff oder eine Alkyl- oder Hydroxyalkylgruppe mit 1 bis einschließlich 6 Kohlenstoffatomen; R₈ und R₉ unabhängig voneinander eine Alkyl- oder eine Hydroxyalkylgruppe mit 1 bis einschließlich 6 Kohlenstoffatomen, A einen Rest eines Glykols oder eines Triols mit 2 bis einschließlich 4 Kohlenstoffatomen; Z ein Anion, ausgewählt aus der Gruppe, bestehend aus Chlorid, Bromid, Methosulfat, Ethosulfat und Gemischen von diesen; m eine Zahl von 1 bis 10 darstellen; Y aus der Gruppe, bestehend aus Wasserstoff, einer Alkylgruppe, einer Hydroxyalkylgruppe, einer Alkylgruppe, entweder substituiert oder unsubstituiert, ausgewählt ist, und worin die Alkyl- oder Hydroxyalkylgruppe 1 bis einschließlich 22 Kohlenstoffatome enthält; und p eine Zahl von 1 bis 3 bedeutet; und
(iii) einem Gemisch davon;
(d) 0,1 bis 3 Gew.-% eines Fettsäureesters, ausgewählt aus der Gruppe, bestehend aus Cetearyloctanoat, Isostearylbenzoat, einem Fett-(C₁₂-C₁₅)-Alkoholbenzoat und deren Gemischen;
(e) 0 bis 5 Gew.-% eines amphoteren Tensids, ausgewählt aus der Gruppe, bestehend aus Cocamidopropyl-Betain, Lauramidopropylbetain, Coco/Oleamidopropylbetain, Cocobetain, Oleylbetain, Cocoamidopropyl-Hydroxysultain, Talgamidopropyl-Hydroxysultain, Dihydroxyethyl-Talgglycinat und deren Gemischen;
(f) 0 bis 5 Gew.-% eines nichtionischen Alkanolamids, ausgewählt aus der Gruppe, bestehend aus Cocamid-MEA, Cocamid-DEA, Soyamid-DEA, Lauramid-DEA, Oleamid-MIPA, Stearamid-MEA, Lauramid-MEA, Capramid-DEA, Ricinolamid-DEA; Myristamid-DEA, Stearamid-DEA, Oleylamid-DEA, Talgamid-DEA, Lauramid-MIPA, Talgamid-MEA, Isostearamid-DEA, Isostearamid-MEA und deren Gemischen; und
(g) einen Träger, enthaltend Wasser.

25. Zusammensetzung nach Anspruch 24, worin die kationische Konditionier-Verbindung aus der Gruppe, bestehend aus Ricinolamidopropyl-Trimoniumchlorid, Ricinolamido-Trimonium-Ethylsulfat, Hydroxy-Stearamidopropyl-Trimonium-Methylsulfat und Hydroxy-Stearamidopropyl-Trimoniumchlorid, Ricinolamidopropyl-Ethyltrimonium-Ethosulfat, Isostearamido-Propyl-Ethyldimonium-Ethosulfat, Quaternium-22, Quaternium-26, Linolamidopropyl-PG-Dimoniumchlorid-Phosphat und deren Gemischen ausgewählt ist.

26. Verfahren zur Behandlung von Haar, wobei dieses gleichzeitig gereinigt und mit Konditioniereigenschaften versehen wird, wonach das Haar mit einer Zusammensetzung nach einem der Ansprüche 1 bis 25 in Berührung gebracht wird; und das Haar anschließend gespült wird.

## Revendications

1. Composition de shampooing conditionnant pour les cheveux, comprenant :
(a) de 1 à 15 % en poids d'un tensio-actif nettoyant anionique ;
(b) de 0,1 à 2 % en poids d'un composé conditionnant cationique polymère ayant une masse moléculaire moyenne d'au moins 100.000 ;
(c) de 0,2 à 10 % en poids d'un tensio-actif conditionnant cationique ;
(d) de 0,1 à 3 % en poids d'un ester gras nonionique ; et
(e) un support comprenant de l'eau.

2. Composition selon la revendication 1, dans laquelle le tensio-actif nettoyant anionique a une chaîne carbonée hydrophobe ayant de 8 à 30 atomes de carbone, limite supérieure comprise, et un fragment hydrophile choisi parmi l'ensemble comprenant les sulfates, les sulfonates, les carbonates, les phosphates, les carboxylates et leurs mélanges.

3. Composition selon la revendication 2, dans laquelle la chaîne carbonée hydrophobe est étherifiée avec une à quatre moles d'oxyde d'éthylène, d'oxyde de propylène ou un de leurs mélanges.

4. Composition selon l'une quelconque des revendications 1 à 3, dans laquelle le tensio-actif nettoyant anionique est un sel d'un métal alcalin, un sel d'ammonium, un sel d'alkylammonium ou un sel d'hydroxyalkylammonium, où le groupe alkyle a de un à trois atomes de carbone, limite supérieure comprise, d'un alkylsulfate, un alkyléthersulfate, un alkyléthersulfonate, un sulfate d'un alkylphénoxyéthanol-polyéthoxylé, un alpha-oléfine sulfonate, un bêta-alkyloxyalcane sulfonate, un alkylarylsulfonate, un alkylcarbonate, un alkyléthercarboxylate, un acide gras, un sulfosuccinate, un sarcosinate, un octoxynolphosphate, un nonoxynolphosphate, un taurate, un tauride gras, un monoglycéride sulfaté, un amidosulfate d'acide gras polyéthoxylé, un isothiènate, ou leurs mélanges, où le fragment gras a de 12 à 18 atomes de carbone, limite supérieure comprise, et le fragment alkyle a de 12 à 18 atomes de carbone, limite supérieure comprise, de préférence un laurylsulfate, un dodécyl-benzènesulfonate, un laurylsulfosuccinate, un lauryl-sulfate éthoxylé ayant de 1 à 4 moles, limite supérieure comprise, d'oxyde d'éthylène, un laurylcarboxylate ayant de 1 à 4 moles d'oxyde d'éthylène, limite supérieure comprise, un laurylsarcosinate, un cocométhyltauride, un amide d'un semi-sulfosuccinate, et leurs mélanges.

5. Composition selon l'une quelconque des revendications 1 à 4, dans laquelle le tensio-actif nettoyant anionique est présent en une quantité comprise entre 3 et 12 %, et de préférence entre 9 et 12 % en poids par rapport à la composition.

6. Composition selon l'une quelconque des revendications 1 à 5, dans laquelle le composé conditionnant cationique polymère a une masse moléculaire moyenne comprise dans l'intervalle de 200.000 à 1.000.000 et de préférence dans l'intervalle de 250.000 à 750.000.

7. Composition selon l'une quelconque des revendications 1 à 6, dans laquelle le composé conditionnant cationique polymère comprend un polymère quaternisé synthétique qui de préférence est choisi parmi l'ensemble comprenant le polyquaternium-1, le polyquaternium-2, le polyquaternium-5, le polyquaternium-6, le polyquaternium-7, le polyquaternium-8, le polyquaternium-9, le polyquaternium-11, le polyquaternium-12, le poly-quaternium-13, le polyquaternium-14, le polyquaternium-15, et leurs mélanges.

8. Composition selon l'une quelconque des revendications 1 à 6, dans laquelle le composé conditionnant cationique polymère comprend un polymère quaternisé d'origine naturelle, qui de préférence est choisi parmi l'ensemble comprenant le polyquaternium-4, le polyquaternium-10, le chlorure de guar-hydroxypropyl-trimonium, et leurs mélanges.

9. Composition selon l'une quelconque des revendications 1 à 8, dans laquelle le composé conditionnant cationique polymère est présent en une quantité comprise dans l'intervalle de 0,2 à 1 % en poids par rapport à la composition.

10. Composition selon la revendication 1, dans laquelle le tensio-actif conditionnant cationique comportant un fragment ester phosphate a la formule développée suivante : 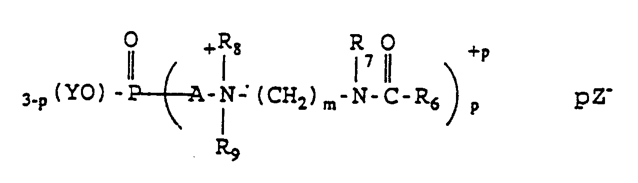 dans laquelle R₆ est un groupe aryle, un groupe alkaryle, un groupe alkyle saturé, un groupe alkyle insaturé, un groupe hydroxyalkyle saturé ou un groupe hydroxyalkyle insaturé, où le groupe alkyle ou hydroxyalkyle a de 7 à 21 atomes de carbone, limite supérieure comprise ; R₇ est un hydrogène, un groupe alkyle ou un groupe hydroxyalkyle ayant de 1 à 6 atomes de carbone, limite supérieure comprise ; R₈ et R₉, indépendamment l'un de l'autre, sont chacun un groupe alkyle ou un groupe hydroxyalkyle ayant de 1 à 6 atomes de carbone, limite supérieure comprise ; a est un résidu d'un glycol ou d'un triol ayant de 2 à 4 atomes de carbone, limite supérieure comprise ; Z est un anion choisi parmi l'ensemble comprenant les anions chlorure, bromure, méthosulfate, éthosulfate et leurs mélanges ; m est un nombre compris entre 1 et 10 ; Y est choisi parmi l'ensemble comprenant l'hydrogène, les groupes alkyle, les groupes hydroxyalkyle et les groupes aryle, où les groupes alkyle ou hydroxyalkyle ont de 1 à 22 atomes de carbone, limite supérieure comprise ; et p est un nombre de 1 à 3, où R₆ est de préférence le fragment alkyle d'un acide gras essentiel, en particulier de l'acide linoléique, de l'acide arachidonique, de l'acide ricinoléique et de leurs mélanges.

11. Composition selon la revendication 10, dans laquelle p vaut 3.

12. Composition selon la revendication 10 ou 11, dans laquelle le tensio-actif cationique est un tri(chlorure de ((alkyle en C₈ à C₂₂)amidopropyl-PG-dimonium), de préférence le phosphate du chlorure de linoléamidopropyle-PG-dimonium.

13. Composition selon l'une quelconque des revendications 1 à 11, dans laquelle le tensio-actif conditionnant cationique est présent en une quantité comprise dans l'intervalle de 0,5 à 5 % en poids par rapport à la composition.

14. Composition selon l'une quelconque des revendications 1 à 13, dans laquelle l'ester gras est présent en une quantité comprise dans l'intervalle de 0,5 à 3 % en poids par rapport à la composition.

15. Composition selon l'une quelconque des revendications 1 à 14, dans laquelle l'ester gras comprend un acide carboxylique ayant de 6 à 12 atomes de carbone, limite supérieure comprise, estérifié par un alcool gras ayant de 12 à 22 atomes de carbone, limite supérieure comprise, qui de préférence est choisi parmi l'ensemble comprenant l'alcool laurylique, l'alcool myristylique, l'alcool cétylique, l'alcool cétéarylique, l'alcool stéarylique, l'alcool isostéarylique, l'alcool oléylique, l'alcool dérivé du suif, l'alcool béhénylique et leurs mélanges.

16. Composition selon la revendication 15, dans laquelle l'ester gras est choisi parmi l'ensemble comprenant l'octanoate de cétyle, l'octanoate de cétéaryle, l'heptanoate de stéaryle, le caprylate de stéaryle, l'octanoate de stéaryle, l'octanoate de lauryle, l'heptanoate de myristyle, l'octanoate d'oléyle et leurs mélanges.

17. Composition selon l'une quelconque des revendications 1 à 14, dans laquelle l'ester gras comprend un acide carboxylique ayant de 8 à 22 atomes de carbone, limite supérieure comprise, estérifié par un alcool ayant de 1 à 6 atomes de carbone, limite supérieure comprise, l'ester gras étant de préférence choisi parmi l'ensemble comprenant le myristate d'isopropyle, le palmitate d'isopropyle, le laurate d'isopropyle, le linoléate d'iso-propyle, l'isostéarate d'isopropyle, l'oléate d'iso-propyle, le stéarate d'isopropyle, le suif-ate d'iso-propyle, le ricinoléate d'isopropyle, le laurate de méthyle, le linoléate de méthyle, le myristate de méthyle, le stéarate de méthyle, le ricinoléate de méthyle, le caprilate de méthyle, l'oléate de méthyle, le palmitate de méthyle, le stéarate de méthyle, le béhénate de méthyle, le soja-ate de méthyle, le suif-ate de méthyle, le béhénate d'isopropyle, le soja-ate d'isopropyle, l'oléate de propyle, l'oléate de butyle, le stéarate de butyle, le coco-ate de méthyle, le saindoux-ate de méthyle, le palmitate d'isobutyle, le myristate de butyle, le palmitate d'éthyle, le myristate d'éthyle, l'oléate d'éthyle, le stéarate d'éthyle, le stéarate d'isobutyle, le myristate d'isobutyle et leurs mélanges.

18. Composition selon l'une quelconque des revendications 1 à 14, dans laquelle l'ester gras comprend un ester de l'acide benzoïque, où l'acide benzoïque est estérifié par un alcool d'estérification ayant de 8 à 22 atomes de carbone, limite supérieure comprise, l'ester de l'acide benzoïque étant de préférence choisi parmi l'ensemble comprenant l'acide benzoïque estérifié par un alcool gras ayant de 12 à 15 atomes de carbone, le benzoate d'isostéaryle, le stéarylbenzoate de PPG-15 éthoxylé et leurs mélanges.

19. Composition selon l'une quelconque des revendications 1 à 18, qui comprend en outre de 0 à 5 % en poids d'un tensio-actif amphotère, de 0 à 5 % en poids d'un tensio-actif nonionique, ou un de leurs mélanges.

20. Composition selon la revendication 19, dans laquelle le tensio-actif amphotère est choisi parmi l'ensemble comprenant les bétaïnes, les hydroxypropylsultaïnes, les oxydes d'amine et leurs mélanges, de préférence parmi l'ensemble comprenant l a coco-amido-propylbétaïne, la lauramidopropylbétaïne, la coco/olé-amidopropylbétaïne, la cocobétaïne, l'oléylbétaïne, la coco-amidopropylhydroxysultaïne, la suif-amidopropylhydroxysultaïne, et le suif-glycinate de dihydroxyéthyle, ou leurs mélanges.

21. Composition selon la revendication 19, dans laquelle le tensio-actif nonionique est choisi parmi l'ensemble comprenant les esters de polyols, les esters de sucres, les alcanolamides d'acides gras, les polyéthylèneglycols, les alcools gras éthoxylés ou propoxylés, les produits de condensation de l'oxyde d'éthylène et d'amides à longue chaîne, et leurs mélanges.

22. Composition selon la revendication 19, dans laquelle le tensio-actif nonionique est un alcanolamide gras choisi parmi l'ensemble comprenant la cocoamide-MEA, la cocoamide-DEA, la sojaamide-DEA, la lauramide-DEA, l'oléamide-MIPA, la stéaramide-MEA, la myristamide-DEA, la stéaramide-DEA, l'oléylamide-DEA, la suifamide-DEA, la lauramide-MIPA, la suifamide-MEA, l'isostéaramide-DEA, l'isostéaramide-MEA et leurs mélanges.

23. Composition selon la revendication 1, dans laquelle le tensio-actif nettoyant anionique est un sel d'un métal alcalin, un sel d'ammonium, un sel d'alkylammonium ou un sel d'hydroxyalkylammonium, où le groupe alkyle a de 1 à 3 atomes de carbone, limite supérieure comprise, d'un alkylsulfate, un alkylsulfate éthoxylé ou un de leurs mélanges ; le composé conditionnant cationique polymère est un chlorure de guar-hydroxypropyltrimonium ; le tensio-actif conditionnant cationique est le chlorure de ricinoléamidopropyltrimonium, le phosphate du chlorure de linoléamidopropyl-PG-dimonium ou un de leurs mélanges ; et l'ester gras est l'octanoate de cétéaryle, cette composition comprenant de préférence encore de 0 à 5 % en poids de cocoamidopropylbétaïne ; de 0 à 5 % en poids d'un alcanolamide gras choisi parmi l'ensemble comprenant la cocoamide-MEA, la linoléamide-DEA et la lauramide-DEA ; ou un de leurs mélanges.

24. Composition de shampooing conditionnant pour les cheveux, comprenant :
(a) de 3 à 15 % en poids d'un tensio-actif nettoyant anionique choisi parmi l'ensemble comprenant le laurylsulfate d'ammonium, le laurylsulfate de sodium, le laurylsulfate d'ammonium éthoxylé à une mole d'oxyde d'éthylène, le laurylsulfate de sodium éthoxylé à une mole d'oxyde d'éthylène et leurs mélanges ;
(b) de 0,1 à 1 % en poids d'un chlorure de guarhydroxypropyltrimonium ;
(c) de 0,2 à 10 % en poids d'un composé conditionnant cationique choisi parmi l'ensemble comprenant :
i) un composé de l'ammonium quaternaire ayant la formule développée suivante : 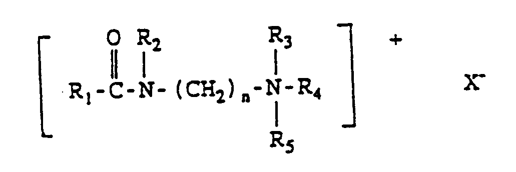 dans laquelle R₁ est un groupe alkyle substitué ou non substitué, saturé ou insaturé, ayant de 5 à 21 atomes de carbone, limite supérieure comprise ; R₂ est un hydrogène ou le radical méthyle ; R₃, R₄ et R₅, indépendamment les uns des autres, sont des radicaux méthyle, éthyle, hydroxyéthyle ou benzyle ; n est un nombre de 1 à 10 ; et X est un anion choisi parmi l'ensemble comprenant les anions chlorure, bromure, éthosulfate, méthosulfate, acétate, nitrate, tosylate, phosphate et leurs mélanges ;
ii) un ester phosphate quaternisé ayant la formule développée suivante : 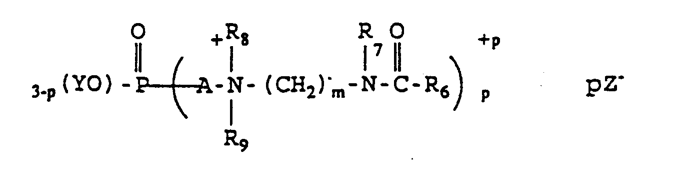 dans laquelle R₆ est un groupe aryle, un groupe alkaryle, un groupe alkyle saturé, un groupe alkyle insaturé, un groupe hydroxyalkyle saturé ou un groupe hydroxyalkyle insaturé, où le groupe alkyle ou hydroxyalkyle a de 7 à 21 atomes de carbone, limite supérieure comprise ; R₇ est un hydrogène, un groupe alkyle ou un groupe hydroxyalkyle ayant de 1 à 6 atomes de carbone, limite supérieure comprise ; R₈ et R₉, indépendamment l'un de l'autre, sont chacun un groupe alkyle ou un groupe hydroxyalkyle ayant de 1 à 6 atomes de carbone, limite supérieure comprise ; A est un résidu d'un glycol ou d'un triol ayant de 2 à 4 atomes de carbone, limite supérieure comprise ; Z est un anion choisi parmi l'ensemble comprenant les anions chlorure, bromure, méthosulfate, éthosulfate et leurs mélanges ; m est un nombre compris entre 1 et 10 ; Y est choisi parmi l'ensemble comprenant l'hydrogène, les groupes alkyle, les groupes hydroxyalkyle et les groupes aryle, où les groupes alkyle ou hydroxyalkyle ont de 1 à 22 atomes de carbone, limite supérieure comprise ; et p est un nombre de 1 à 3 ; et
iii) un de leurs mélanges ;
(d) de 0,1 à 3 % en poids d'un ester gras choisi parmi l'ensemble comprenant l'octanoate de cétéaryle, le benzoate d'isostéaryle, un (alcool gras en C₁₂ à C₁₅) benzoate et un de leurs mélanges ;
(e) de 0 à 5 % en poids d'un tensio-actif amphotère, choisi parmi l'ensemble comprenant la coco-amidopropylbétaïne, la lauramidopropylbétaïne, la coco/oléamidopropylbétaïne, la cocobétaïne, l'oléylbétaïne, la cocoamidopropylhydroxysultaïne, la suif-amidopropylhydroxysultaïne, et le suif-gycinate de dihydroxyéthyle, ou leurs mélanges ;
(f) de 0 à 5 % en poids d'un alcanolamide nonionique, choisi parmi l'ensemble comprenant la cocoamide-MEA, la cocoamide-DEA, la sojaamide-DEA, la lauramide-DEA, l'oléamide-MIPA, la stéaramide-MEA, la myristamide-MEA, la lauramide-MEA, la capramide-DEA, la ricinoamide-DEA, la myristamide-DEA, la stéaramide-DEA, l'oléylamide-DEA, la suifamide-DEA, la lauramide-MIPA, la suifamide-MEA, l'isostéaramide-DEA, l'isostéaramide-MEA et leurs mélanges ; et
(g) un support comprenant de l'eau.

25. Composition selon la revendication 24, dans laquelle le composé conditionnant cationique est choisi parmi l'ensemble comprenant le chlorure de ricinoléamido-propyl trimonium, l'éthylsulfate de ricinoléamido trimonium, le méthylsulfate d'hydroxystéaramidopropyl trimonium et le chlorure d'hydroxystéréamidopropyl trimonium, l'éthosulfate de ricinoléamidopropyléthyl dimonium, l'éthosulfate d'isostéréamidopropyléthyl dimonium, le quaternium-22, le quaternium-26, le phosphate du chlorure de linoléamidopropyl-PG-dimonium et leurs mélanges.

26. Procédé pour traiter les cheveux, pour simultanément nettoyer les cheveux et leur conférer des propriétés conditionnantes, qui consiste à mettre les cheveux en contact avec une composition selon l'une quelconque des revendications 1 à 25, puis à rincer les cheveux.
